(19) Europäisches Patentamt
European Patent Office
Office européen des brevets

(11) Veröffentlichungsnummer: **0 209 701**
**B1**

(12) # EUROPÄISCHE PATENTSCHRIFT

(45) Veröffentlichungstag der Patentschrift: **16.08.89**

(51) Int. Cl.⁴: **C 07 D 211/90**, A 61 K 31/44, C 07 D 491/04, C 07 C 49/12 // (C07D491/048, 307:00, 221:00)

(21) Anmeldenummer: **86107843.4**

(22) Anmeldetag: **09.06.86**

(54) **Neue 1,4-Dihydropyridine, Verfahren zur Herstellung und ihre Verwendung in Arzneimitteln.**

(30) Priorität: **19.06.85 DE 3521761**

(43) Veröffentlichungstag der Anmeldung: **28.01.87 Patentblatt 87/5**

(45) Bekanntmachung des Hinweises auf die Patenterteilung: **16.08.89 Patentblatt 89/33**

(84) Benannte Vertragsstaaten: **AT BE CH DE FR GB IT LI NL SE**

(56) Entgegenhaltungen:
**EP-A- 0 088 274**

**CHEMICAL ABSTRACTS, Band 103, 1985, Seite 700, Zusammenfassung Nr. 160398j, Columbus, Ohio, US; & JP-A-60 56 956**
**CHEMICAL ABSTRACTS, Band 98, 1983, Seite 536, Zusammenfassung Nr. 89135s, Columbus, Ohio, US; V.V. KASTRON et al.: "Synthesis and pharmacological activity of 4-aryl-1,4-dihydropyridines"**

**Die Akte enthält technische Angaben, die nach dem Eingang der Anmeldung eingereicht wurden und die nicht in dieser Patentschrift enthalten sind.**

(73) Patentinhaber: **BAYER AG, D-5090 Leverkusen 1 Bayerwerk (DE)**

(72) Erfinder: **Goldmann, Siegfried, Dr., Am Osterholz 91, D-5600 Wuppertal 11 (DE)**
Erfinder: **Stoltefuss, Jürgen, Dipl.-Ing., Parkstrasse 20, D-5657 Haan (DE)**
Erfinder: **Franckowiak, Gerhard, Dr., Henselweg 10, D-5600 Wuppertal 1 (DE)**
Erfinder: **Gross, Rainer, Dr., Platzhofstrasse 23, D-5600 Wuppertal 1 (DE)**
Erfinder: **Schramm, Matthias, Dr., Paffrather Strasse 38, D-5000 Köln 80 (DE)**
Erfinder: **Thomas, Günter, Dr. Bayer Italia S.p.A., Rep. Pharmacologis Via delle Groane 126, I-200024 Garbagnate Milano (IT)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue 1,4-Dihydropyridine, Verfahren zu ihrer Herstellung sowie ihre Verwendung in Arzneimitteln, insbesondere in kreislaufbeeinflussenden Mitteln.

Es ist bereits bekannt, daß 1,4-Dihydropyridine gefäßerweiternde Eigenschaften bsitzen und als Coronarmittel und Antihypertensiva verwendet werden können (vgl. Brit. Patent 1 173 062 und 1 358 951; DE-OS 2 629 892 und 2 752 820). Weiterhin ist bekannt, daß 1,4-Dihydropyridine eine Hemmung der Kontraktionskraft von glatten und cardialen Muskeln bewirken und zur Behandlung von Coronar- und Gefäßerkrankungen eingesetzt werden können [vgl. Fleckenstein, Ann. Rev. Pharmacol. Toxicol. *17*, 149-166 (1977)].

Aus Chemical Abstracts Vol. 103 (1985), Seite 200, Nr. 160398j sind Dihydropyridine bekannt, die in 3- und 5-Position Estergruppen tragen. Diese Diester zeigen calciumantagonistische Wirkungen, während die erfindungsgemäßen Verbindungen anstelle einer Estergruppe eine Nitro-, Cyano- oder Lactongruppe enthalten. Überraschenderweise wird durch diese Änderung der Struktur eine signifikante Wirkungsänderung erzielt. Während die bekannten Diester eine signifikante Senkung der Kontraktivität am Vorhof des Meerschweinchenherzens zeigen, bewirken die erfindungsgemäßen Verbindungen eine deutliche Steigerung. Im Gegensatz zu der calciumantagonistischen Wirkung der bekannten Dihydropyridine zeigen die erfindungsgemässen Dihydropyridine eine calciumagonistische Wirkung.

Bei Kenntnis der Eigenschaften der bekannten Dihydropyridine war nicht vorhersehbar, daß die erfindungsgemäßen Verbindungen dieser Stoffklassen eine kontraktionskraftverstärkende, am Herzmuskel positiv inotrope Wirkung besitzen.

Die Erfindung betrifft neue 1,4-Dihydropyridine der allgemeinen Formel (I),

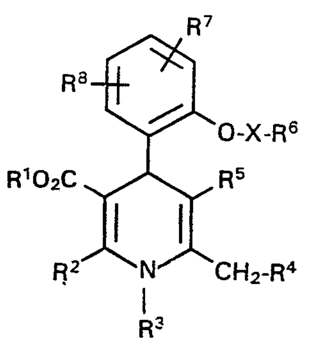

(I)

in welcher

$R^1$ für geradkettiges, verzweigtes oder cyclisches Alkyl oder Alkenyl mit bis zu 10 C-Atomen steht, das gegebenenfalls durch bis zu 3 Sauerstoff- und/oder Schwefelatome in der Kette unterbrochen ist und das gegebenenfalls substituiert ist durch Phenyl, Cyan, Hydroxy, ein oder mehrere Fluor, Chlor, Brom, Carboxy, $C_1$-$C_4$ -Alkoxycarbonyl, $C_1$-$C_4$-Alkylsulfonyl oder durch eine Aminogruppe, wobei die Aminogruppe einen oder zwei gleiche oder verschiedene Substituenten aus der Gruppe $C_1$-$C_4$-Alkyl, Phenyl, Benzyl, Acetyl oder Benzoyl tragen kann,

$R^2$ für geradkettiges oder verzweigtes Alkyl mit bis zu 4 C-Atomen steht, das gegebenenfalls substituiert ist durch ein oder mehrere Fluor, Chlor, Brom, Phenyl oder Hydroxy, oder
für Cyano steht,

$R^3$ für Wasserstoff oder
für geradkettiges oder verzweigtes Alkyl mit bis zu 4 C-Atomen steht,

$R^4$ für Wasserstoff, Hydroxy,
für geradkettiges oder verzweigtes Alkyl mit bis zu 4 C-Atomen,
für Fluor, Chlor, Brom , oder
für eine Gruppe O-Y steht,
wobei
Y für Acetyl, Methoxymethyl oder Benzyl steht,

$R^5$ für Nitro,
für Cyano oder
für die Gruppe $CO_2R^9$ steht,
wobei
$R^9$ und $R^4$ gemeinsam eine direkte Bindung bedeuten,

X für $\rangle C=O$, $SO_2$- oder -CO-NH- steht,

$R^6$ für geradkettiges, verzweigtes oder cyclisches Alkyl oder Alkenyl mit bis zu 18 C-Atomen steht, das gegebenenfalls substituiert ist durch Phenyl, ein oder mehrere Fluor, Chlor, Nitro, Carboxy oder $C_1$-$C_4$-Alkoxycarbonyl, oder
für gegebenenfalls durch Fluor, Chlor, Brom oder $C_1$-$C_4$-Alkyl substituiertes Thienyl, Furyl, Pyrryl, Pyridyl, Pyrimidyl, Pyrazinyl, Pyrolyl, Pyridazinyl steht, oder
für $C_6$-$C_{10}$-Aryl steht, das gegebenenfalls substituiert ist durch bis zu 4 gleiche oder verschiedene Substituenten der Reihe Fluor, Chlor, Brom, Nitro, $C_1$-$C_6$-Alkyl, $C_1$-$C_3$-Alkoxy, $C_1$-$C_3$-Alkylthio, $C_1$-$C_4$-Alkoxycarbonyl, $C_1$-$C_4$-Alkylsulfonyl, Trifluormethoxy, Trifluormethylthio, Trifluormethyl oder Amino, wobei diese Aminogruppe einen oder zwei Substituenten aus der Reihe Benzoyl, Acetyl, Phenyl oder Methyl tragen kann oder

für den Rest $-CH_2$ 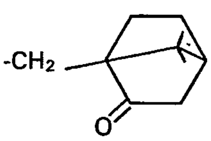 steht,

und

$R^7$, $R^8$ gleich oder verschieden sind und
für Wasserstoff,
für $C_1$-$C_6$-Alkoxy,
für Fluor, Chlor, Brom,
für $C_1$-$C_6$-Alkyl,
für Nitro,
für Cyan oder
für Trifluormethyl steht,

sowie deren Salze.

Bevorzugt sind Verbindungen der allgemeinen Formel I, in welcher

$R^1$ für geradkettiges, verzweigtes oder cyclisches Alkyl oder Alkenyl mit bis zu 8 C-Atomen steht, das gegebenenfalls durch ein Sauerstoff- oder Schwefelatom in der Kette unterbrochen ist und das gegebenenfalls substituiert ist durch Phenyl, Cyan, ein oder mehrere Fluor oder N-Benzyl-N--methyl-amino,

$R^2$ für Methyl, Hydroxymethyl oder Ethyl, für Cyano steht,

$R^3$ für Wasserstoff oder für Methyl oder Ethyl steht,

$R^4$ für Wasserstoff, Hydroxy, für Methyl, Ethyl, für Chlor, Brom oder für eine Gruppe O-Y steht, worin Y für Acetyl steht,

$R^5$ für Nitro oder für $CO_2R^9$ steht, wobei $R^9$ und $R^4$ gemeinsam eine direkte Bindung bedeuten,

Y für $>C=O$, $-SO_2-$ oder -CO-NH- steht,

$R^6$ für geradkettiges, verzweigtes oder cyclisches Alkyl oder Alkenyl mit bis zu 16 C-Atomen steht, das gegebenenfalls substituiert ist durch Phenyl oder ein oder mehrere Fluor oder Chlor, für gegebenenfalls durch Chlor oder Methyl substituiertes Thienyl, Furyl oder Pyridyl steht, oder für Phenyl oder Naphthyl steht, das gegebenenfalls bis zu 3 gleiche oder verschiedene Substituenten aus der Reihe Fluor, Chlor, Nitro, $C_1$-$C_4$-Alkyl, Methoxy, Methylthio, Trifluormethoxy, Trifluormethylthio, Trifluormethyl oder Acetylamino trägt, oder

für den Rest 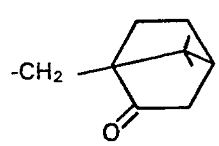 steht,

und

$R^7$, $R^8$ gleich oder verschieden sind und für Wasserstoff, für $C_1$-$C_4$-Alkoxy, für Chlor für $C_1$-$C_4$-Alkyl, für Nitro oder für Trifluormethyl steht,

sowie deren Salze.

Die erfindungsgemäßen Stoffe können in Form ihrer Salze vorliegen. Im allgemeinen sind dies Salze der erfindungsgemäßen Stoffe mit anorganischen oder organischen Säuren. Bevorzugt sind jedoch die physiologisch unbedenklichen Salze der erfindungsgemässen Stoffe mit anorganischen und organischen Säuren. Als Beispiele seien genannt: Hydrohalogenide, Hydrogensulfate, Sulfate, Hydrogenphosphate, Acetate, Maleate, Citrate, Fumarate, Tartrate, Lactate oder Benzoate.

Die erfindungsgemäßen Verbindungen der Formel I, in welcher $R^1$-$R^8$ und X die oben angegebene Bedeutung haben, $R^5$ jedoch nicht für den Rest $CO_2R^9$ stehen darf, erhält man, wenn man

[A] Aldehyde der allgemeinen Formel (II)

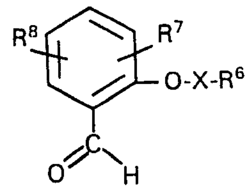 (II),

in welcher $R^6$, $R^7$, $R^8$ und X die oben angegebene Bedeutung haben und Ketoverbindungen der allgemeinen Formel (III)

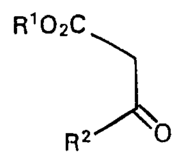 (III),

in welcher $R^1$ und $R^2$ die oben angegebene Bedeutung haben, mit Ketoverbindungen der allgemeinen Formel (IV)

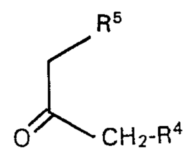 (IV),

in welcher $R^4$ und $R^5$ die oben angegebene Bedeutung haben und Aminen der allgemeinen Formel (V)

$$R^3\text{-}NH_2 \qquad (V),$$

in welcher $R^3$ die oben angegebene Bedeutung hat, gegebenenfalls in Gegenwart von Wasser und/oder inerten organischen Lösungsmitteln umsetzt,

oder wenn man

[B] Aldehyde der allgemeinen Formel (II) mit Ketoverbindungen der allgemeinen Formel (III) und Enaminen der allgemeinen Formel (VI)

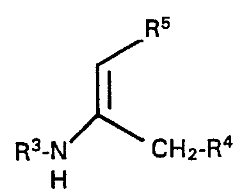 (VI),

in welcher $R^3$, $R^4$ und $R^5$ die oben angegebene Bedeutung haben, gegebenenfalls in Gegenwart von Wasser und/oder inerten organischen Lösungsmitteln umsetzt,

oder wenn man

[C] Aldehyde der allgemeinen Formel (II) mit Ketoverbindungen der allgemeinen Formel (IV) und Enaminen der allgemeinen Formel (VII)

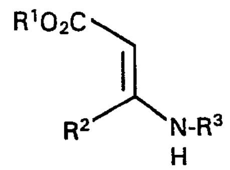

(VII),

in welcher $R^1$, $R^2$ und $R^3$ die oben angegebene Bedeutung haben, gegebenenfalls in Gegenwart von Wasser und/oder inerten organischen Lösungsmitteln umsetzt,

oder wenn man

[D] Ketoverbindungen der allgemeinen Formel (III) mit Aminen der allgemeinen Formel (V) und Ylidenverbindungen der allgemeinen Formel (VIII)

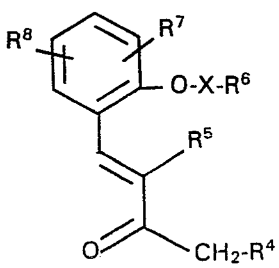

(VIII),

in welcher $R^4$, $R^5$, $R^6$, $R^7$, $R^8$ und X die oben angegebene Bedeutung haben, gegebenenfalls in Gegenwart von Wasser und/oder inerten organischen Lösungsmitteln umsetzt,

oder wenn man

[E] Ketoverbindungen der allgemeinen Formel (IV) mit Aminen der allgemeinen Formel (V) und Ylidenverbindungen der allgemeinen Formel (IX)

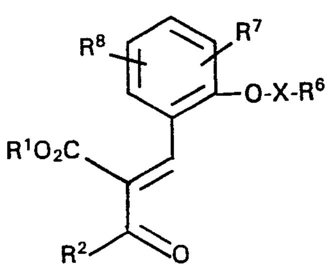

(IX),

in welcher $R^1$, $R^2$, $R^6$, $R^7$, $R^8$ und X die oben angegebene Bedeutung haben, gegebenenfalls in Gegenwart von Wasser und/oder inerten organischen Lösungsmitteln umsetzt,

oder wenn man

[F] Ylidenverbindungen der allgemeinen Formel (VIII) mit Enaminen der allgemeinen Formel (VII) gegebenenfalls in Gegenwart von Wasser und/oder inerten organischen Lösungsmitteln umsetzt,

oder wenn man

[G] Ylidenverbindungen der allgemeinen Formel (IX) mit Enaminen der allgemeinen Formel (VI) gegebenenfalls in Gegenwart von Wasser und/oder inerten organischen Lösungsmitteln umsetzt.

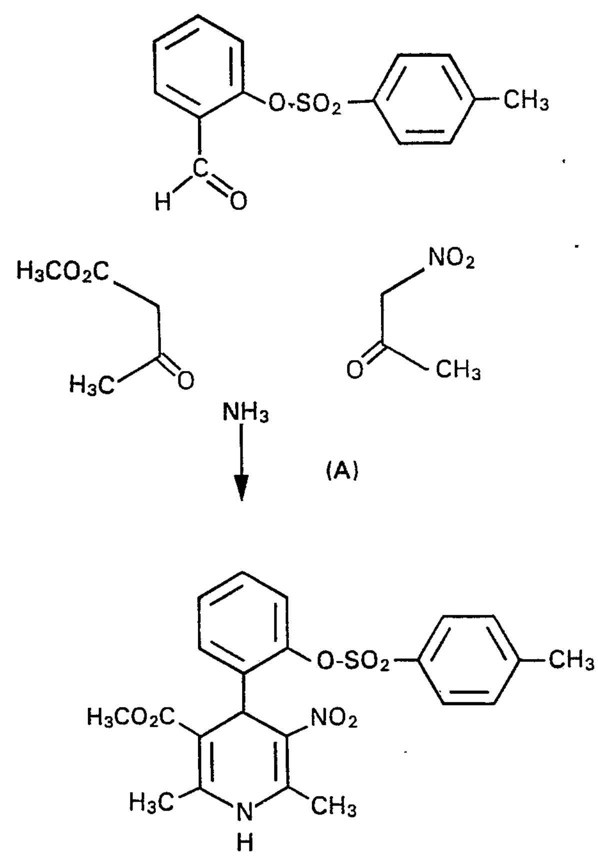


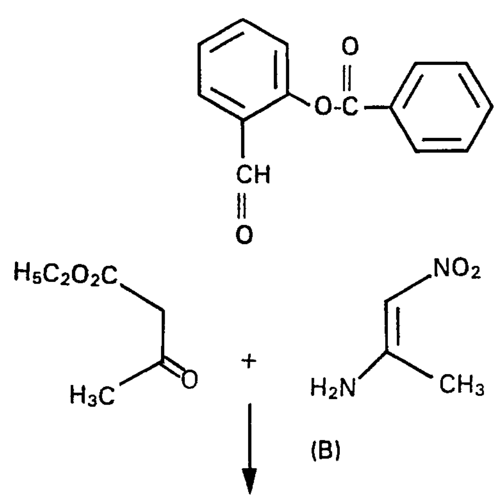


(B)

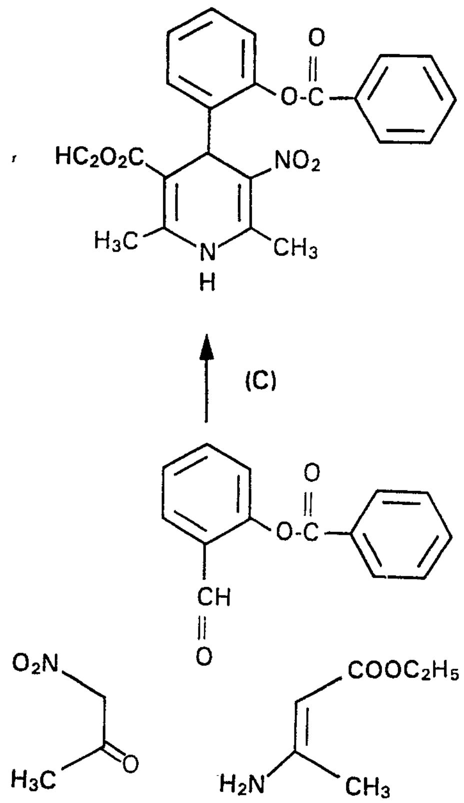
(C)

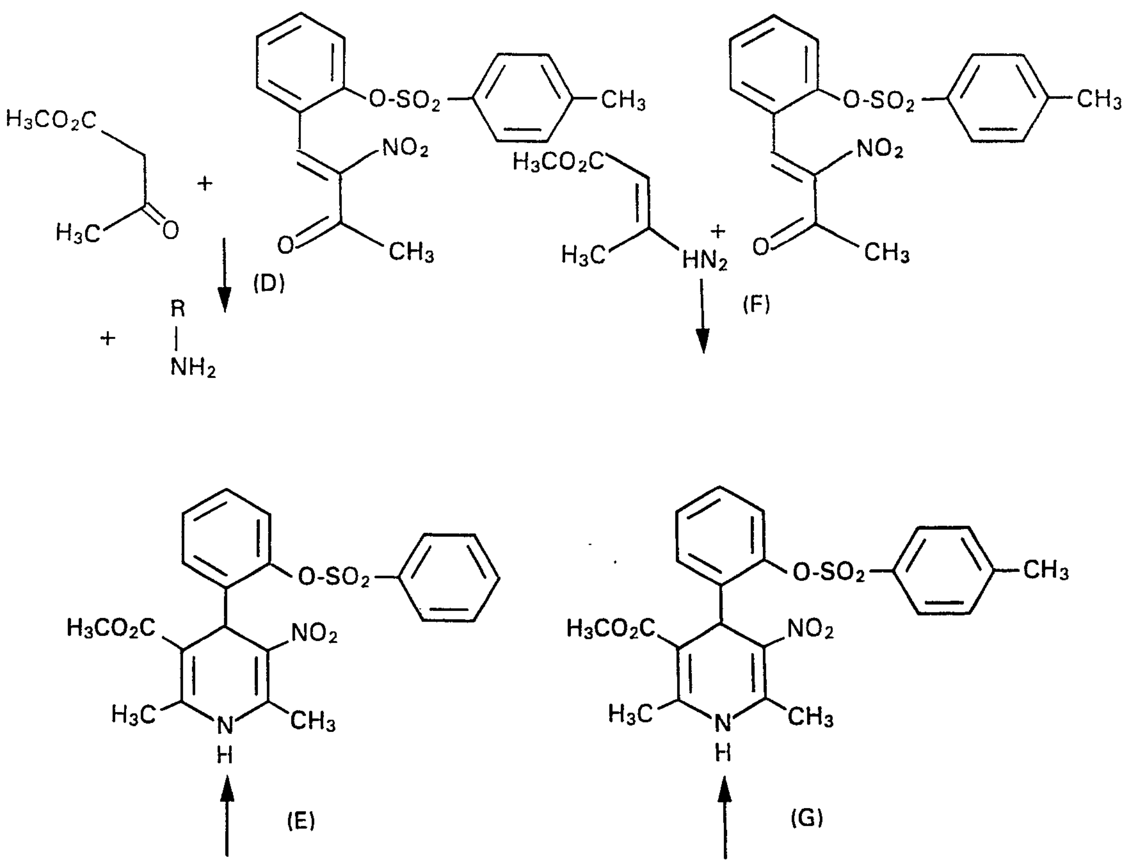
(D)
(F)
(E)
(G)

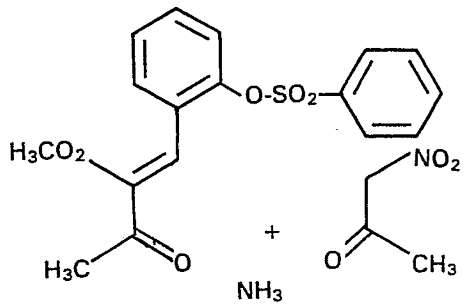

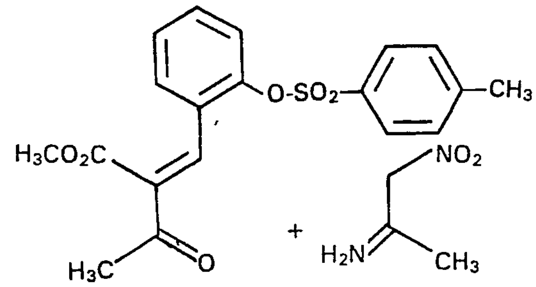

Die als Ausgangsstoffe verwendeten Aldehyde der allgemeinen Formel II sind bekannt oder können nach literaturbekannten Methoden hergestellt werden [vgl. W.J. Dale, H.E. Hennis J. Am. Chem. Soc. *78*, 2543 (1956)].

Die als Ausgangsstoffe verwendeten Ketoverbindungen III und IV sind bekannt oder können nach literaturbekannten Methoden hergestellt werden [vgl. D. Borrmann in Houben-Weyl «Methoden der organischen Chemie» VII/4, 230 ff. (1968); P. Pollet, S. Gelin, Tetrahedron *34*, 1453 (1978); Y. Oikawa, K. Sugano, O. Yonemitsu, J. Org. Chem. *43*, 2087 (1978); C.W. Scaife, J. Chem. Soc. (London) *1946*, 1100; C.D. Hurd, M.E. Nilson, J. Org. Chem. *20*, 927 (1955)].

Verbindungen der Formel V sind bekannt und können käuflich erworben werden.

Die als Ausgangsstoffe verwendeten Enamine VI und VII sind bekannt oder können nach literaturbekannten Methoden hergestellt werden [vgl. S.A. Glickman, A.C. Cope, J. Am. Chem. Soc. *67*, 1017 (1945); H. Böhme, K.-H. Weisel, Arch. Pharm. *310*, 30 (1977)].

Die als Ausgangsstoffe verwendeten Ylidenverbindungen VIII und IX sind bekannt oder können nach literaturbekannten Methoden hergestellt werden [vgl. G. Jones «The Knoevenagel Condensation», Organic Reactions XV, 204 ff. (1967); für $R^5 = NO_2$ vgl. W. Sassenberg, A. Dornow, Liebigs Ann. Chem. *602*, 14 (1957)].

Als Verdünnungsmittel kommen für die Verfahren A bis G alle inerten organischen Lösungsmittel infrage. Hierzu gehören bevorzugt Alkohole wie Methanol, Ethanol, n- bzw. iso-Propanol, Ether wie Diethylether, Tetrahydrofuran, Dioxan oder Glykolmono- oder Diethylether, Eisessig, Pyridin, Dimethylformamid, Dimethylsulfoxid, Acetonitril oder Hexamethylphosphorsäuretriamid.

Die Reaktionstemperaturen für Verfahren A bis G können in einem größeren Bereich variiert werden. Im allgemeinen arbeitet man in einem Bereich von 10°C bis 200°C, bevorzugt von 20°C bis 150°C.

Die Umsetzung kann bei Normaldruck, aber auch bei erhöhtem Druck durchgeführt werden. Im allgemeinen arbeitet man bei Normaldruck.

Bei der Durchführung der erfindungsgemäßen Verfahren ist das Verhältnis der an der Reaktion beteiligten Stoffe beliebig. Im allgemeinen arbeitet man jedoch mit molaren Mengen der Reaktanden.

Bevorzugte Verfahren zur Darstellung der Verbindungen I, in welchen $R^1$ bis $R^8$ und X die angegebene Bedeutung haben, $R^5$ jedoch nicht für die Gruppe $CO_2R^9$ stehen darf, sind die Verfahren B, C, F und G. Besonders bevorzugt sind die Verfahren B und C.

Die erfindungsgemäßen Verbindungen der allgemeinen Formel I, in welcher

$R^1$-$R^3$, $R^6$-$R^8$ und X die angegebene Bedeutung haben, $R^5$ für die Gruppe $CO_2R^9$ steht und $R^4$ und $R^9$ gemeinsam eine Bindung darstellen, erhält man, wenn man

[H] Aldehyde der allgemeinen Formel II

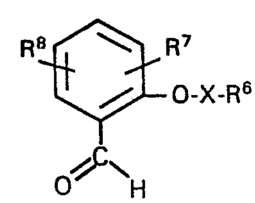

(II),

in welcher $R^6$-$R^8$ und X die oben angegebene Bedeutung haben, mit Ketoverbindungen der allgemeinen Formel III

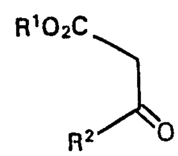

(III),

in welcher $R^1$ und $R^2$ die oben angegebene Bedeutung haben, mit Enaminen der Formel X

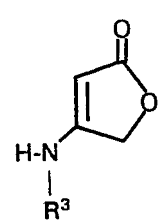

(X),

in welcher $R^3$ die oben angegebene Bedeutung hat, gegebenenfalls in Anwesenheit von Wasser und/oder inerten organischen Lösungsmitteln umsetzt,

oder wenn man

[I] Dihydropyridine der allgemeinen Formel XI

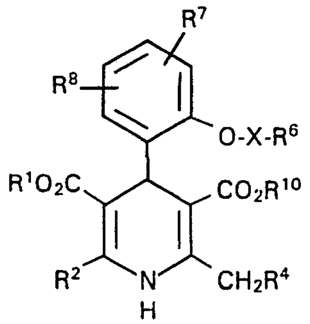

(XI),

in welcher

$R^1$-$R^3$, $R^6$-$R^8$ und X die oben angegebenen Bedeutung haben,

$R^4$ für Halogen
für eine Gruppe O-Y steht,
wobei
Y die oben angegebene Bedeutung hat,
und
$R^{10}$ für geradkettiges oder verzweigtes Alkyl mit bis zu 6 C-Atomen steht,

gegebenenfalls in Gegenwart inerter organischer Lösungsmittel und gegebenenfalls in Anwesenheit von Basen cyclisiert,
oder
wenn $R^4$ für Halogen steht, mit oder ohne Lösungsmittel pyrolisiert.

Je nach Art der verwendeten Ausgangsstoffe kann die Synthese der Stoffe I mit Verfahren H oder I durch folgende Schemata verdeutlicht werden:

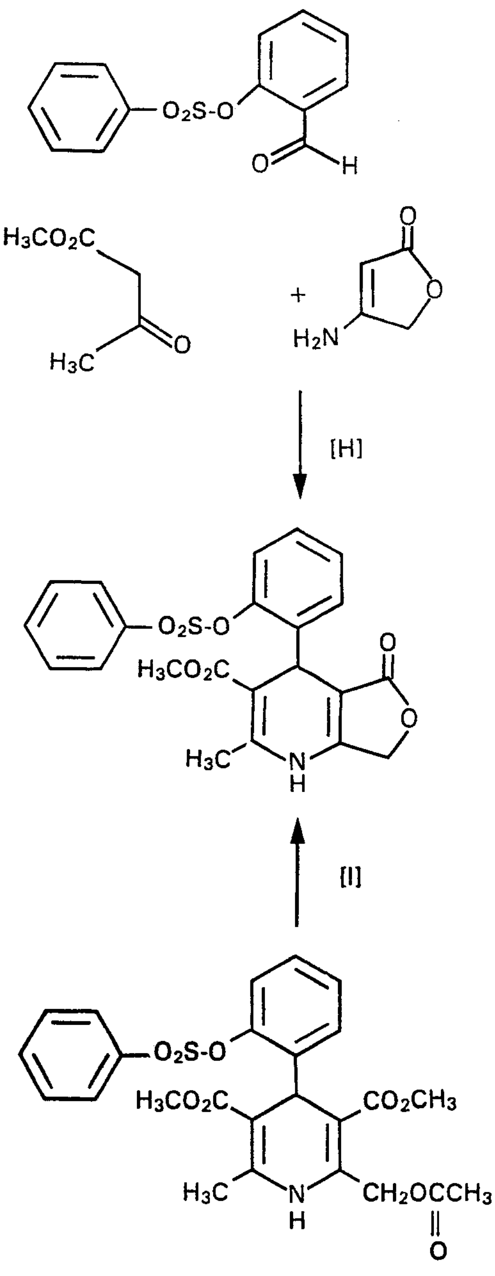


(XI)

Die als Ausgangsstoffe verwendeten Verbindungen II, III und X sind bekannt oder können nach bekannten Methoden hergestellt werden (vgl. Literaturzitate und EP 123 095).

Die Reaktionsbedingungen wie Lösungsmittel, Temperaturen und Mengenverhältnisse sind bei Verfahren H die gleichen wie bereits für Verfahren A bis G angegeben.

Die Ausgangsverbindungen der Formel XI sind neu und werden nach Verfahren A bis G wie unter Punkt 1) angegeben, hergestellt.

Als Basen eignen sich die üblichen Basen wie zum Beispiel Alkali- oder Erdalkalihydroxide, besonders Natrium-, Kalium-, Calciumhydroxid oder Amine wie Ammoniak, Triethylamin, Pyridin. Die Cyclisierung kann in den üblichen Lösungsmitteln wie aromatische Kohlenwasserstoffe (z.B. Benzol, Toluol) Alkoholen (Ethanol, Propanol, Methanol) oder Essigsäure durchgeführt werden. Die Cyclisierung erfolgt bei Temperaturen von 10°C bis 200°C, bevorzugt bei 20°C bis 150°C.

Die Pyrolyse kann mit oder ohne Lösungsmittel durchgeführt werden. Als Lösungsmittel kommen gegebenenfalls alle üblichen inerten organischen Lösungsmittel infrage. Dazu zählen bevorzugt Kohlenwasserstoffe wie Benzol, Toluol oder Xylol, Tetralin, Erdölfraktionen, Ether wie Diethylether, Dioxan, Tetrahydrofuran, Glykolmono- bzw. Diethylether, Halogenkohlenwasserstoffe wie Di-, Tri- oder Tetrachlormethan, Dichlor- oder Trichlorethylen.

Die Pyrolyse wird in einem Temperaturbereich von 20°C bis 300°C, bevorzugt von 40°C bis 250°C durchgeführt.

Die Pyrolyse kann bei normalem, erhöhtem oder erniedrigten Druck durchgeführt werden. Im allgemeinen arbeitet man bei Normaldruck.

Bevorzugt ist das Verfahren I.

Die vorstehenden Herstellungsverfahren sind lediglich zur Verdeutlichung angegeben. Die Herstellung der Verbindungen der Formel I ist nicht auf diese Verfahren beschränkt, sondern jede Modifikation dieser Verfahren ist in gleicher Weise für die Herstellung der erfindungsgemäßen Verbindungen anwendbar.

Die erfindungsgemäßen Verbindungen existieren in stereoisomeren Formen, die sich entweder wie Bild und Spiegelbild (Enantiomere) oder die sich nicht wie Bild und Spiegelbild (Diastereomerengemische ) verhalten. Die Erfindung betrifft sowohl die Antipoden als auch die Racemformen sowie Diastereomerengemische. Die Racemformen lasse sich ebenso wie die Diastereomeren in bekannter Weise in die stereoisomer einheitliche Bestandteile trennen (vgl. E.L. Eliel, Stereochemistry of Carbon Compounds, McGraw Hill, 1962).

Die erfindungsgemäßen Verbindungen haben eine positiv inotrope und koronardilatierende Wirkung und zeigen somit ein nicht vorhersehbares und wertvolles pharmakologisches Wirkspektrum. Sie können als kraislaufbeeinflussende Mittel, als Koronartherapeutika, Antiarrhythmika, zur Behandlung von Herzinsuffizienz und zur Beeinflussung des Blutzuckerspiegels eingesetzt werden.

Die Herzkontraktilität-verstärkende Wirkung wurde an isolierten Vorhöfen von Meerschweinchenherzen gefunden.

Dazu werden die linken Vorhöfe von Meerschweinchenherzen isoliert und in ein thermostatisiertes Organbad gehängt, welches eine isotonische Mineralsalzlösung, die dem Ionenmilieu und dem pH-Wert von Körperflüssigkeiten angepaßt ist, mit geeigneten Nährstoffen enthält. Dieses Organbad wurde mit einem Gasgemisch bestehend aus Sauerstoff und Kohlendioxid begast, wobei der Kohlendioxidgehalt so bemessen ist, daß der pH-Wert des Organbades konstant bleibt. Die linken Vorhöfe wurden in das Organbad eingespannt, die Spannung mittels eines Kraftaufnehmers registriert, wobei ein bestimmter Grundtonus eingestellt wird. Anschließend wurden die linken Vorhöfe kontinuierlich in bestimmten Abständen elektrisch gereizt und die dabei erfolgenden Kontraktionen registriert. Nach Zugabe des Wirkstoffs wurden die Kontraktionen weiterhin registriert.

| Beispiel-Nr. | Kontrakt.-Steigerung bei $10^{-5}$ g/ml [%] |
|---|---|
| 2 | + 165 |
| 6 | + 50 |
| 7 | + 10 |
| 12 | + 78 |
| 14 | + 15 |
| 15 | + 33 |
| 30 | + 16 |
| 31 | + 32 |
| 33 | + 37 |

Die koronardilatierende Wirkung wurde am isoliert perfundierten Meerschweinchenherzen gefunden.

Dazu wurden die Herzen von 250 bis 350 g schweren Albino Meerschweinchen verwendet. Die Tiere werden mit einem Schlag auf den Kopf gelötet, der Thorax geöffnet, in die freipräparierte Aorta eine Metallkanüle eingebunden und der linke Vorhof geöffnet. Das Herz wurde mit den Lungen aus dem Thorax herausgetrennt und über die Aortakanüle an die Perfusionsapparatur bei laufender Perfusion angeschlossen. Die Lungen wurden an den Lungenwurzeln abgetrennt. Als Perfusionsmedium diente Krebs-Henseleit-Lösung (2) (118,5 mmol/l NaCl, 4,75 mmol/l KCl, 1,19 mmol/l $KH_2PO_4$, 1,19 mmol/l, $MgSO_4$, 25 mmol/l $NaHCO_3$, 0,013 mmol/l NaEDTA), deren $CaCl_2$ je nach Bedarf variiert wurde, in der Regel jedoch 1,2 mmol/l Glucose betrug.

Als energielieferndes Substrat wurden 10 mmol/l Glucose zugesetzt. Vor der Perfusion wurde die Lösung partikelfrei filtriert. Die Lösung wurde mit Carbogen (95% $O_2$, 5% $CO_2$) zur Aufrechterhaltung des pH-Wertes von 7,4 begast. Die Herzen wurden mit konstantem Fluß (10 ml/min) bei 32°C mittels einer Rollenquetschpumpe perfundiert.

Zur Messung der Herzfunktion wurde ein flüssigkeitsgefüllter Latexballon, der über eine Flüssigkeitssäule mit einem Druckaufnehmer verbunden war, durch den linken Vorhof in den linken Ventrikel eingeführt. Der Perfusionsdruck wurde mittels eines

Druckaufnehmers, der vor dem Herzen mit dem Perfusionssystem in Verbindung stand, registriert. Unter diesen Bedingungen zeigte eine Senkung des Perfusionsdrucks eine Koronardilatation.

Die erfindungsgemäßen Verbindungen wurden in geeigneten Verdünnungen in das Perfusionssystem kurz vor den isolierten Herzen infundiert.

| Beispiel-Nr. | Widerstandserniedrigung bei $10^{-6}$ g/ml [%] |
|---|---|
| 2 | – 39 |
| 6 | – 23 |
| 7 | – 8 |
| 12 | – 21 |
| 14 | – 33 |
| 15 | – 55 |
| 30 | – 21 |
| 31 | – 83 |
| 33 | – 50 |

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. Erdnuß/Sesamöl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester), Polyoxyethylen-Fettalkohol-Ether (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspension und/oder Elixieren, die für orale Anwendung gedacht sind, können die Wirkstoffe außer den obengenannten Hilfsstoffen mit verschiedenen Geschmackaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg, Körpergewicht.

Trotzdem kann es gegebenenfalls erforderlich sein, von den genannten Mengen abzuweichen, und zwar in Abhängigkeit vom Körpergewicht des Versuchstieres bzw. der Art der Applikation, aber auch aufgrund der Tierart und deren individuellem Verhalten gegenüber dem Medikament bzw. deren Art von dessen Formulierung und dem Zeitpunkt bzw. Intervall, zu weichem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese Applikation in der Humanmedizin ist der gleiche Dosierungsspielraum vorgesehen. Sinngemäß gelten hierbei auch die obigen Ausführungen.

*Herstellungsbeispiele*

*Beispiel 1*

2-Methyl-5-oxo-4-(2-phenylsulfonyloxy)phenyl--1,4,5,7-tetrahydrofuro[3,4-b]pyridin-3-carbonsäureethylester

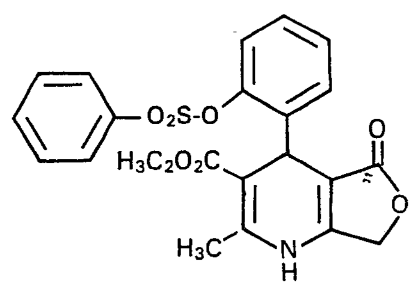

a) Je 20 mmol 2-(2-Phenylsulfonyloxy)benzaldehyd, β-Aminocrotonsäureethylester und γ-Chloracetessigsäureethylester werden in Ethanol unter Rückfluß gekocht. Anschließend wird die Lösung eingeengt und der Rückstand in Dichlormethan aufgenommen. Die organische Phase wird mit Wasser gewaschen, getrocknet und eingedampft. Der Rückstand kristallisiert mit Methanol.

b) Je 20 mmol 2-(Phenylsulfonyloxy)benzalde-

hyd, β-Aminocrotonsäureethylester und γ-Acetoxyacetessigsäureethylester werden über Nacht in Ethanol gekocht, anschließend mit KOH versetzt und weitere 15 min gekocht.

Die Mischung wird eingeengt, mit Wasser versetzt und mit Dichlormethan ausgeschüttelt. Die organische Phase wird getrocknet und eingeengt. Der Rückstand kristallisiert mit Methanol.

Schmelzpunkt: 96-100°C.

*Beispiel 2*

1,4-Dihydro-2,6-dimethyl-5-Nitro-4-(2-p-tolylsulfonyloxy)-phenyl-pyridin-3-carbonsäuremethylester

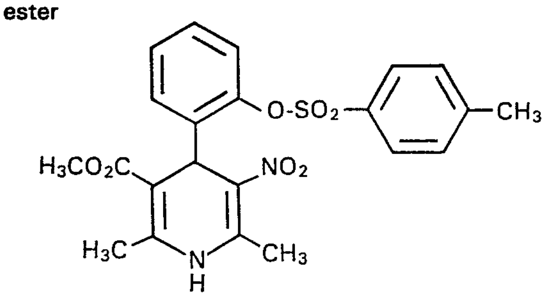

Je 20 mmol Nitroaceton, β-Aminocrotonsäuremethylester und 2-(p-Toluolsulfonyloxy)benzaldehyd werden in 50 ml Ethanol 3 h gekocht, die Lösung eingeengt und der Rückstand an Kieselgel mit Toluol/Essigester = chromatographiert.

Schmelzpunkt: 184-187°C.

*Beispiel 3*

Je 40 mmol 2-(4-Nitrobenzyloxy)benzaldehyd, Nitroaceton und β-Aminocrotonsäuremethylester werden in 60 ml Ethanol 2 h zum Rückfluss erhitzt. Beim Abkühlen des Reaktionsgemisches tritt Kristallisation ein. Es wird abgesaugt und mit Ethanol gewaschen. Man erhält 3,9 g (21,5%) gelbe Kristalle.

Schmelzpunkt: 245°C.

Analog den oben beschriebenen Verfahren wurden die in den folgenden Tabellen aufgeführten Beispiele erhalten:

TABELLE 1

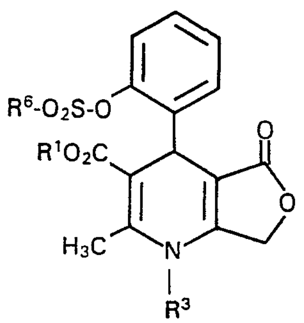

| Bsp.-Nr. | $R^1$ | $R^3$ | $R^6$ | Schmelzpunkt [°C] |
|---|---|---|---|---|
| 4 | $CH_2CH_3$ | H | 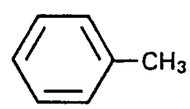 | 121-3 |
| 5 | $CH_3$ | H | 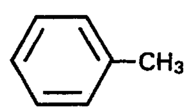 | 175-8 |
| 6 | $CH_2CH_3$ | H | $(CH_2)_4Cl$ | amorph. |
| 7 | $CH_2CH_3$ | H | 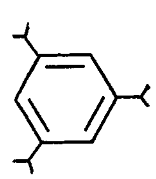 | 218-20 |

TABELLE 1 (Fortsetzung)

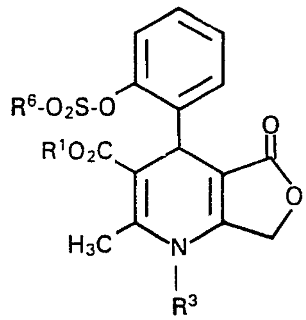

| Bsp.-Nr. | $R^1$ | $R^3$ | $R^6$ | Schmelzpunkt [°C] |
|---|---|---|---|---|
| 8 | $CH_2CH_3$ | H | 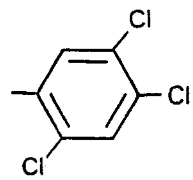 | 245-7 |
| 9 | $CH_3$ | H | 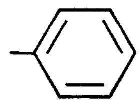 | 201-5 |
| 10 | $CH_3$ | $CH_3$ | 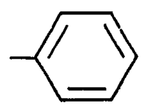 | 165-9 |
| 11 | $(CH_2)_3CH_3$ | H | 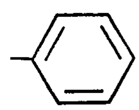 | 88-91 |

TABELLE 2

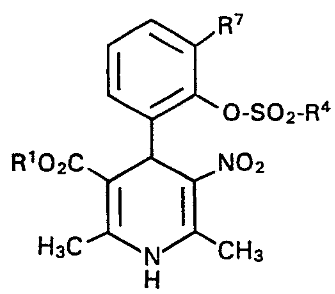

| Bsp.-Nr. | $R^1$ | $R^6$ | $R^7$ | Schmelzpunkt [°C] |
|---|---|---|---|---|
| 12 | $CH_2CH_3$ | 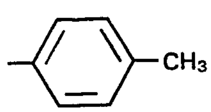 | H | 53-4 |

TABELLE 2 (Fortsetzung)

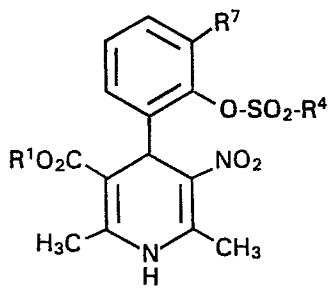

| Bsp.-Nr. | $R^1$ | $R^6$ | $R^7$ | Schmelzpunkt [°C] |
|---|---|---|---|---|
| 13 | $(CH_2)_2OCH_3$ | 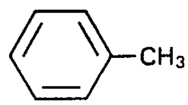 | H | 210-2 |
| 14 | $(CH_2)_3CH_3$ | 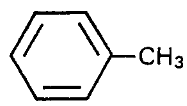 | H | 176-8 |
| 15 | $CH_2$-$CH=CH_2$ | 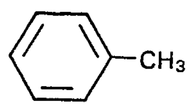 | H | amorph |
| 16 | $(CH_2)_7CH_3$ | 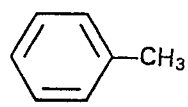 | H | Öl |
| 17 | $CH_3$ | 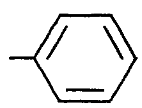 | H | 220-5 |
| 18 | $CH_2CH_3$ | 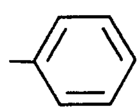 | H | 127-9 |
| 19 | $CH_3$ | 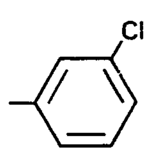 | H | 70-4 |
| 20 | $CH_2CH_3$ | 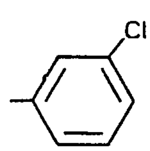 | H | amorph |

TABELLE 2 (Fortsetzung)

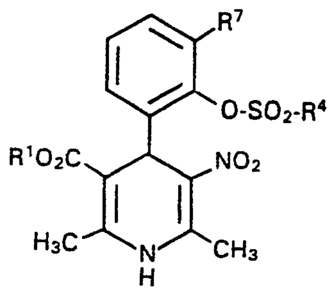

| Bsp.-Nr. | $R^1$ | $R^6$ | $R^7$ | Schmelzpunkt [°C] |
|---|---|---|---|---|
| 21 | $CH_2CH_3$ | 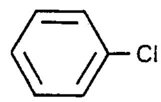 | H | 121-5 |
| 22 | $CH_3$ | 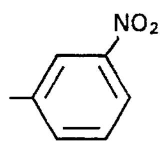 | H | 96-9 |
| 23 | $CH_2CH_3$ | 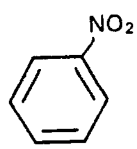 | H | 72-80 |
| 24 | $CH_3$ | 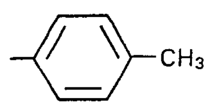 | $OCH_3$ | 190-210 |
| 25 | $CH_2CH_3$ | 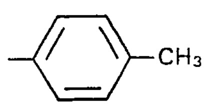 | $OCH_3$ | 169-87 |
| 26 | $CH_3$ | 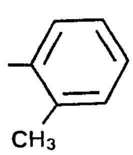 | H | 210-6 |
| 27 | $CH_2CH_3$ | 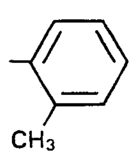 | H | 278-85 |
| 28 | $CH_3$ | $(CH_2)_{15}CH_3$ | H | 126 |

TABELLE 2 (Fortsetzung)

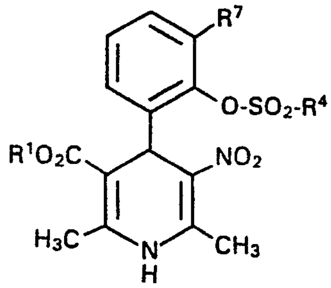

| Bsp.-Nr. | $R^1$ | $R^6$ | $R^7$ | Schmelzpunkt [°C] |
|---|---|---|---|---|
| 29 | $CH_2CH_3$ | $(CH_2)_{15}CH_3$ | H | Öl |
| 30 | $CH_3$ | 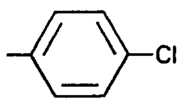 | H | 210-6 |
| 31 | $CH_3$ | 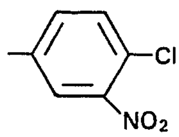 | H | amorph |
| 32 | $CH_2CH_3$ | 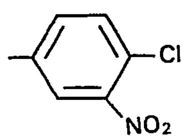 | H | amorph |
| 33 | $CH_2CH_3$ | 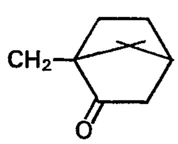 | H | amorph |
| 34 | $CH_2CH_3$ | 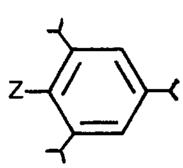 | H | 107-9 |
| 35 | $CH_2CH_3$ | 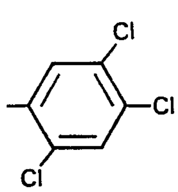 | H | 94-7 |

TABELLE 2 (Fortsetzung)

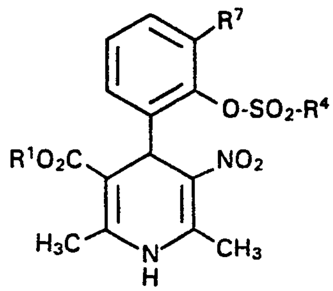

| Bsp.-Nr. | $R^1$ | $R^6$ | $R^7$ | Schmelzpunkt [°C] |
|---|---|---|---|---|
| 36 | $CH_3$ | 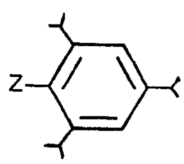 | H | 114 |
| 37 | $CH_3$ | 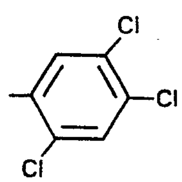 | H | 120 |
| 38 | $CH_2CH_3$ | 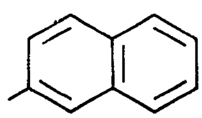 | H | 170-2 |
| 39 | $CH_2CH_3$ | 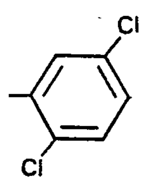 | H | 108-11 |
| 40 | $CH_3$ | 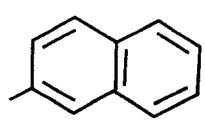 | H | 156-60 |
| 41 | $CH_3$ | 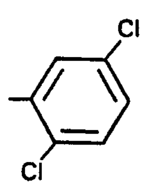 | H | 135-40 |
| 42 | $CH_2$ 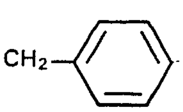 | 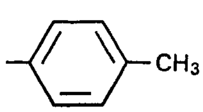 | H | amorph |

TABELLE 2 (Fortsetzung)

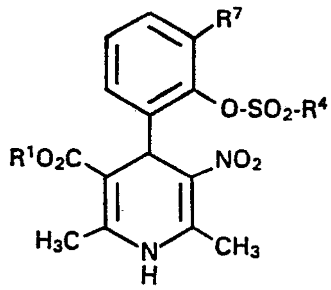

| Bsp.-Nr. | $R^1$ | $R^6$ | $R^7$ | Schmelzpunkt [°C] |
|---|---|---|---|---|
| 43 | 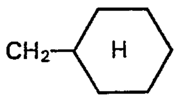 | 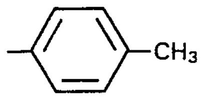 | H | amorph |
| 44 | $CH_2CH_3$ | 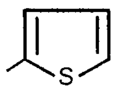 | H | Schaum |
| 45 | $CH_2CH_3$ | 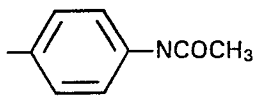 | H | 233 |
| 46 | $CH_3$ | 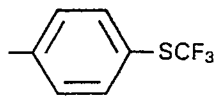 | H | Schaum |
| 47 | $CH_3$ | 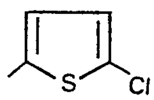 | H | Schaum |

TABELLE 3

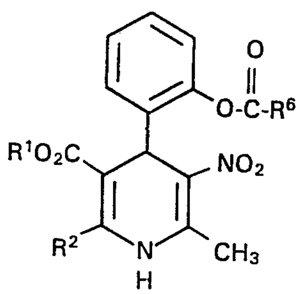

| Bsp.-Nr. | $R^1$ | $R^2$ | $R^6$ | Schmelzpunkt [°C] |
|---|---|---|---|---|
| 48 | $CH_2CH_3$ | $CH_3$ | 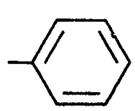 | 180-1 |

TABELLE 3 (Fortsetzung)

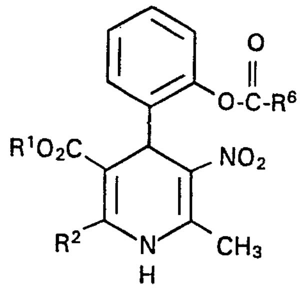

| Bsp.-Nr. | $R^1$ | $R^2$ | $R^6$ | Schmelzpunkt [°C] |
|---|---|---|---|---|
| 49 | $CH_3$ | Ch3 | 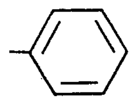 | 190-3 |
| 50 | $CH_3$ | $CH_2CH_3$ | 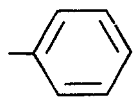 | 198-200 |
| 51 | $C_4H_9$ | $CH_3$ | 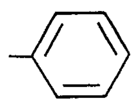 | 175-8 |
| 52 | $CH_2CH_3$ | $CH_3$ | 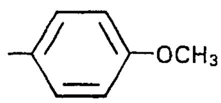 | 146 |
| 53 | $CH_3$ | $CH_3$ | 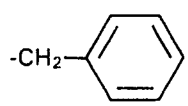 | 140 |
| 54 | $CH_3$ | $CH_2CH_3$ | 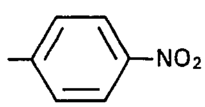 | 237 |
| 55 | $CH_3$ | $CH_3$ | 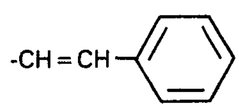 | 222 |

TABELLE 4

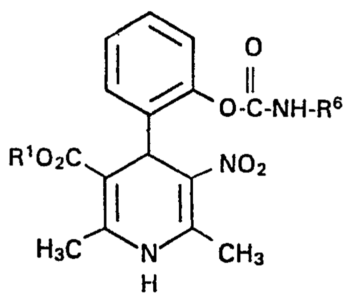

| Bsp.-Nr. | $R^1$ | $R^6$ | Schmelzpunkt [°C] |
|---|---|---|---|
| 56 | $CH_3$ | 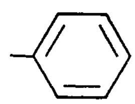 | 144-50 |
| 57 | $CH_2CH_3$ | 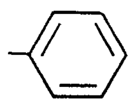 | 227-33 |

*Beispiel 58*

1,4-Dihydro-2,6-dimethyl-4-(2-benzoyloxy-3--methoxy-phenyl)-5-nitro-pyridin-3-carbonsäure-ethylester vom Schmp.: 195°C

*Beispiel 59*

1,4-Dihydro-2,6-dimethyl-4-(2-benzolsulfonyl-oxyphenyl)-5-nitro-pyridon-3-carbonsäureethyl-ester vom Schmp.: 222°C.

**Patentansprüche**

1. Dihydropyridine der allgemeinen Formel I

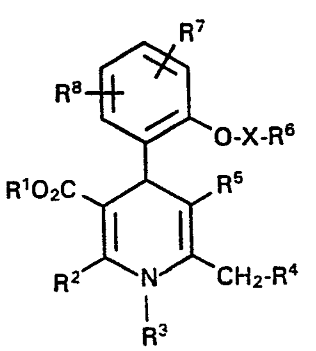

(I)

in welcher

$R^1$ für geradkettiges, verzweigtes oder cyclisches Alkyl oder Alkenyl mit bis zu 10 C-Atomen steht, das gegebenenfalls durch bis zu 3 Sauerstoff- und/oder Schwefelatome in der Kette unterbrochen ist und das gegebenenfalls substituiert ist durch Phenyl, Cyan, Hydroxy, ein oder mehrere Fluor, Chlor, Brom, Carboxy, $C_1$-$C_4$ -Alkoxycarbonyl, $C_1$-$C_4$-Alkylsulfonyl oder durch eine Aminogruppe, wobei die Aminogruppe einen oder zwei gleiche oder verschiedene Substituenten aus der Gruppe $C_1$-$C_4$-Alkyl, Phenyl, Benzyl, Acetyl oder Benzoyl tragen kann,

$R^2$ für geradkettiges oder verzweigtes Alkyl mit bis zu 4 C-Atomen steht, das gegebenenfalls substituiert ist durch ein oder mehrere Fluor, Chlor, Brom, Phenyl oder Hydroxy, oder
für Cyano steht,

$R^3$ für Wasserstoff oder
für geradkettiges oder verzweigtes Alkyl mit bis zu 4 C-Atomen steht,

$R^4$ für Wasserstoff, Hydroxy,
für geradkettiges oder verzweigtes Alkyl mit bis zu 4 C-Atomen,
für Fluor, Chlor, Brom , oder
für eine Gruppe O-Y steht,
wobei
Y für Acetyl, Methoxymethyl oder Benzyl steht,

$R^5$ für Nitro,
für Cyano oder
für die Gruppe $CO_2R^9$ steht,
wobei
$R^9$ und $R^4$ gemeinsam eine direkte Bindung bedeuten,

X für $>C=O$, $SO_2$- oder -CO-NH- steht,

$R^6$ für geradkettiges, verzweigtes oder cyclisches Alkyl oder Alkenyl mit bis zu 18 C-Atomen steht, das gegebenenfalls substituiert ist durch Phenyl, ein oder mehrere Fluor, Chlor, Nitro, Carboxy oder $C_1$-$C_4$-Alkoxycarbonyl, oder
für gegebenenfalls durch Fluor, Chlor, Brom oder $C_1$-$C_4$-Alkyl substituiertes Thienyl, Furyl, Pyrryl, Pyridyl, Pyrimidyl, Pyrazinyl, Pyrolyl, Pyridazinyl steht, oder
für $C_6$-$C_{10}$-Aryl steht, das gegebenenfalls substituiert ist durch bis zu 4 gleiche oder verschiedene Substituenten der Reihe Fluor, Chlor, Brom, Nitro, $C_1$-$C_6$-Alkyl, $C_1$-$C_3$-Alkoxy, $C_1$-$C_3$-Alkylthio, $C_1$-$C_4$-Alkoxycarbonyl, $C_1$-$C_4$-Alkylsulfonyl, Trifluormethoxy, Trifluormethylthio, Trifluormethyl oder Amino, wobei diese Aminogruppe einen oder zwei Substituenten aus der Reihe Benzoyl, Acetyl, Phenyl oder Methyl tragen kann oder

für den Rest 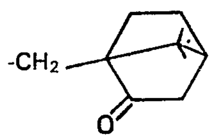 steht,

und
$R^7$, $R^8$ gleich oder verschieden sind und
für Wasserstoff,
für $C_1$-$C_6$-Alkoxy,
für Fluor, Chlor, Brom,
für $C_1$-$C_6$-Alkyl,
für Nitro,
für Cyan oder
für Trifluormethyl steht,

sowie deren Salze.

2. Verbindungen gemäß Anspruch 1, in welchen
$R^1$ für geradkettiges, verzweigtes oder cyclisches Alkyl oder Alkenyl mit bis zu 8 C-Atomen steht, das gegebenenfalls durch ein Sauerstoff- oder Schwefelatom in der Kette unterbrochen ist und das gegebenenfalls substituiert ist durch Phenyl, Cyan, ein oder mehrere Fluor oder N-Benzyl-N-methyl-amino,
$R^2$ für Methyl, Hydroxymethyl oder Ethyl, für Cyano steht,
$R^3$ für Wasserstoff oder für Methyl oder Ethyl steht,
$R^4$ für Wasserstoff, Hydroxy,
für Methyl, Ethyl,
für Chlor, Brom oder
für eine Gruppe O-Y steht,
worin
Y für Acetyl steht,
$R^5$ für Nitro oder
für $CO_2R^9$ steht,
wobei
$R^9$ und $R^4$ gemeinsam eine direkte Bindung bedeuten,
X für $>C=O$, $-SO_2-$ oder -CO-NH- steht,
$R^6$ für geradkettiges, verzweigtes oder cyclisches Alkyl oder Alkenyl mit bis zu 16 C-Atomen steht, das gegebenenfalls substituiert ist durch Phenyl oder ein oder mehrere Fluor oder Chlor,
für gegebenenfalls durch Chlor oder Methyl substituiertes Thienyl, Furyl oder Pyridyl steht, oder
für Phenyl oder Naphthyl steht, das gegebenenfalls bis zu 3 gleiche oder verschiedene Substituenten aus der Reihe Fluor, Chlor, Nitro, $C_1$-$C_4$-Alkyl, Methoxy, Methylthio, Trifluormethoxy, Trifluormethylthio, Trifluormethyl oder Acetylamino trägt, oder

für den Rest 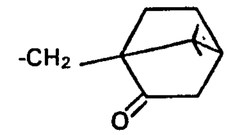 steht,

und
$R^7$, $R^8$ gleich oder verschieden sind und
für Wasserstoff,
für $C_1$-$C_4$-Alkoxy,
für Chlor
für $C_1$-$C_4$-Alkyl,
für Nitro oder
für Trifluormethyl steht,

sowie deren Salze.

3. Dihydropyridine gemäß Ansprüchen 1 und 2 zur Bekämpfung von Kreislauferkrankungen, Koronarerkrankungen, Herzrhythmusstörungen, zur Behandlung von Herzinsuffizienz und/oder zur Beeinflussung des Blutzuckerspiegels.

4. Arzneimittel enthaltend als Wirkstoff Dihydropyridine gemäß Ansprüchen 1 und 2.

5. Verfahren zur Herstellung von Dihydropyridinen der allgemeinen Formel I

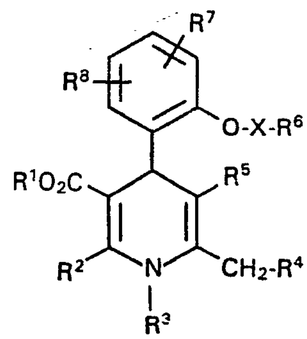 (I)

in welcher

$R^1$ für geradkettiges, verzweigtes oder cyclisches Alkyl oder Alkenyl mit bis zu 10 C-Atomen steht, das gegebenenfalls durch bis zu 3 Sauerstoff- und/oder Schwefelatome in der Kette unterbrochen ist und das gegebenenfalls substituiert ist durch Phenyl, Cyan, Hydroxy, ein oder mehrere Fluor, Chlor, Brom, Carboxy, $C_1$-$C_4$ -Alkoxycarbonyl, $C_1$-$C_4$-Alkylsulfonyl oder durch eine Aminogruppe, wobei die Aminogruppe einen oder zwei gleiche oder verschiedene Substituenten aus der Gruppe $C_1$-$C_4$-Alkyl, Phenyl, Benzyl, Acetyl oder Benzoyl tragen kann,

$R^2$ für geradkettiges oder verzweigtes Alkyl mit bis zu 4 C-Atomen steht, das gegebenenfalls substituiert ist durch ein oder mehrere Fluor, Chlor, Brom, Phenyl oder Hydroxy, oder für Cyano steht,

$R^3$ für Wasserstoff oder für geradkettiges oder verzweigtes Alkyl mit bis zu 4 C-Atomen steht,

$R^4$ für Wasserstoff, Hydroxy, für geradkettiges oder verzweigtes Alkyl mit bis zu 4 C-Atomen, für Fluor, Chlor, Brom , oder für eine Gruppe O-Y steht, wobei Y für Acetyl, Methoxymethyl oder Benzyl steht,

$R^5$ für Nitro, für Cyano oder für die Gruppe $CO_2R^9$ steht, wobei $R^9$ und $R^4$ gemeinsam eine direkte Bindung bedeuten,

X für $>C=O$, $SO_2$- oder -CO-NH- steht,

$R^6$ für geradkettiges, verzweigtes oder cyclisches Alkyl oder Alkenyl mit bis zu 18 C-Atomen steht, das gegebenenfalls substituiert ist durch Phenyl, ein oder mehrere Fluor, Chlor, Nitro, Carboxy oder $C_1$-$C_4$-Alkoxycarbonyl, oder für gegebenenfalls durch Fluor, Chlor, Brom oder $C_1$-$C_4$-Alkyl substituiertes Thienyl, Furyl, Pyrryl, Pyridyl, Pyrimidyl, Pyrazinyl, Pyrolyl, Pyridazinyl steht, oder für $C_6$-$C_{10}$-Aryl steht, das gegebenenfalls substituiert ist durch bis zu 4 gleiche oder verschiedene Substituenten der Reihe Fluor, Chlor, Brom, Nitro, $C_1$-$C_6$-Alkyl, $C_1$-$C_3$-Alkoxy, $C_1$-$C_3$-Alkylthio, $C_1$-$C_4$-Alkoxycarbonyl, $C_1$-$C_4$-Alkylsulfonyl, Trifluormethoxy, Trifluormethylthio, Trifluormethyl oder Amino, wobei diese Aminogruppe einen oder zwei Substituenten aus der Reihe Benzoyl, Acetyl, Phenyl oder Methyl tragen kann oder

für den Rest $-CH_2$ 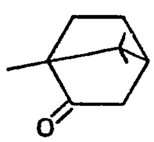 steht,

und

$R^7$, $R^8$ gleich oder verschieden sind und für Wasserstoff, für $C_1$-$C_6$-Alkoxy, für Fluor, Chlor, Brom, für $C_1$-$C_6$-Alkyl, für Nitro, für Cyan oder für Trifluormethyl steht,

sowie deren Salze,

wobei $R^5$ jedoch nicht für den Rest $CO_2R^9$ stehen darf, dadurch gekennzeichnet, daß man

[A] Aldehyde der allgemeinen Formel (II)

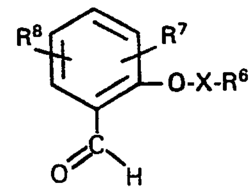 (II),

in welcher $R^6$, $R^7$, $R^8$ und X die oben angegebene Bedeutung haben und Ketoverbindungen der allgemeinen Formel (III)

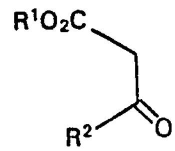 (III),

in welcher $R^1$ und $R^2$ die oben angegebene Bedeutung haben, mit Ketoverbindungen der allgemeinen Formel (IV)

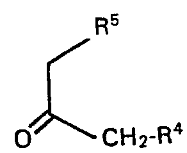 (IV),

in welcher $R^4$ und $R^5$ die oben angegebene Bedeutung haben und Aminen der allgemeinen Formel (V)

$R^3$-$NH_2$ (V),

in welcher $R^3$ die oben angegebene Bedeutung hat, gegebenenfalls in Gegenwart von Wasser und/oder inerten organischen Lösungsmitteln umsetzt,

oder wenn man

[B] Aldehyde der allgemeinen Formel (II) mit Ketoverbindungen der allgemeinen Formel (III) und Enaminen der allgemeinen Formel (VI)

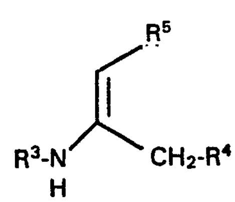 ((VI),

in welcher $R^3$, $R^4$ und $R^5$ die oben angegebene Bedeutung haben, gegebenenfalls in Gegenwart von Wasser und/oder inerten organischen Lösungsmitteln umsetzt,

oder wenn man

[C] Aldehyde der allgemeinen Formel (II) mit Ketoverbindungen der allgemeinen Formel (IV) und Enaminen der allgemeinen Formel (VII)

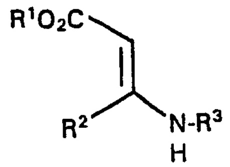

(VII),

in welcher $R^1$, $R^2$ und $R^3$ die oben angegebene Bedeutung haben, gegebenenfalls in Gegenwart von Wasser und/oder inerten organischen Lösungsmitteln umsetzt,

oder wenn man

[D] Ketoverbindungen der allgemeinen Formel (III) mit Aminen der allgemeinen Formel (V) und Ylidenverbindungen der allgemeinen Formel (VIII)

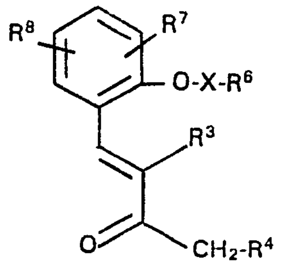

(VIII),

in welcher $R^4$, $R^5$, $R^6$, $R^7$, $R^8$ und X die oben angegebene Bedeutung haben, gegebenenfalls in Gegenwart von Wasser und/oder inerten organischen Lösungsmitteln umsetzt,

oder wenn man

[E] Ketoverbindungen der allgemeinen Formel (IV) mit Aminen der allgemeinen Formel (V) und Ylidenverbindungen der allgemeinen Formel (IX)

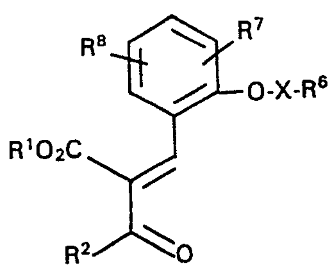

(IX),

in welcher $R^1$, $R^2$, $R^6$, $R^7$, $R^8$ und X die oben angegebene Bedeutung haben, gegebenenfalls in Gegenwart von Wasser und/oder inerten organischen Lösungsmitteln umsetzt,

oder wenn man

[F] Ylidenverbindungen der allgemeinen Formel (VIII) mit Enaminen der allgemeinen Formel (VII) gegebenenfalls in Gegenwart von Wasser und/oder inerten organischen Lösungsmitteln umsetzt,

oder wenn man

[G] Ylidenverbindungen der allgemeinen Formel (IX) mit Enaminen der allgemeinen Formel (VI) gegebenenfalls in Gegenwart von Wasser und/oder inerten organischen Lösungsmitteln umsetzt.

6. Verfahren zur Herstellung von Dihydropyridinen der allgemeinen Formel I, gemäß Anspruch 5, in welcher $R^1$, $R^2$, $R^3$, $R^4$, $R^5$, X, $R^6$, $R^7$ und $R^8$ die in Anspruch 5 angegebene Bedeutung haben, wobei $R^5$ für die Gruppe $CO_2R^9$ steht und $R^4$ und $R^9$ gemeinsam eine Bindung darstellen, dadurch gekennzeichnet, daß man

[H] Aldehyde der allgemeinen Formel II

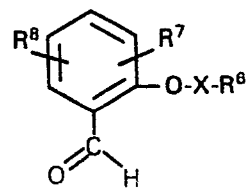

(II),

in welcher $R^6$-$R^8$ und X die oben angegebene Bedeutung haben, mit Ketoverbindungen der allgemeinen Formel III

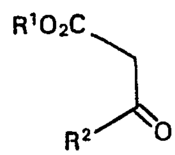

(III),

in welcher $R^1$ und $R^2$ die oben angegebene Bedeutung haben, mit Enaminen der Formel X

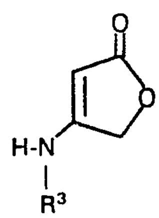

(X),

in welcher $R^3$ die oben angegebene Bedeutung hat, gegebenenfalls in Anwesenheit von Wasser und/oder inerten organischen Lösungsmitteln umsetzt,

oder daß man

[I] Dihydropyridine der allgemeinen Formel XI

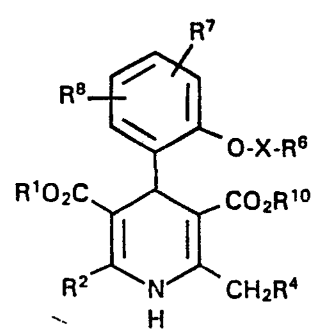

(XI),

in welcher

$R^4$ für Halogen
für eine Gruppe O-Y steht,
wobei
Y die oben angegebene Bedeutung hat,

und

$R^{10}$ für geradkettiges oder verzweigtes Alkyl mit bis zu 6 C-Atomen steht,

gegebenenfalls in Gegenwart inerter organischer Lösungsmittel und gegebenenfalls in Anwesenheit von Basen cyclisiert,
oder
wenn $R^4$ für Halogen steht, mit oder ohne Lösungsmittel pyrolisiert.

7. Verfahren zur Herstellung von Arzneimitteln gemäß Anspruch 4, dadurch gekennzeichnet, daß man die Wirkstoffe mit üblichen Hilfs- und/oder Trägerstoffen mischt.

8. Verbindung von Dihydropyridinen der Ansprüche 1 und 2 zur Herstellung von Arzneimitteln zur Bekämpfung von Kreislauferkrankungen, Koronarerkrankungen, Herzrhythmusstörungen, zur Behandlung von Herzinsuffizienz und/oder zur Beeinflussung des Blutzuckerspiegels.

## Claims

1. Dihydropyridines of the general formula I

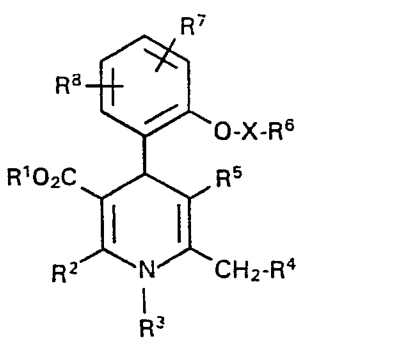

(I)

in which

$R^1$ represents straight-chain, branched or cyclic alkyl or alkenyl having up to 10 C atoms, which is optionally interrupted in the chain by up to 3 oxygen and/or sulphur atoms and is optionally substituited by phenyl, cyano, hydroxyl, one or more fluorine, chlorine, bromine, carboxyl, $C_1$-$C_4$-alkoxycarbonyl, $C_1$-$C_4$-alkylsulphonyl, or by an amino group, it being possible for the amino group to carry one or two identical or different substituents from the group comprising $C_1$-$C_4$-alkyl, phenyl, benzyl, acetyl or benzoyl,
$R^2$ represents straight-chain or branched alkyl having up to 4 C atoms, which is optionally substituted by one or more fluorine, chlorine, bromine, phenyl or hydroxyl, or represents cyano,
$R^3$ represents hydrogen, or
represents straight-chain or branched alkyl having up to 4 C atoms,
$R^4$ represents hydrogen or hydroxyl,
represents straight-chain or branched alkyl having up to 4 C atoms,
represents fluorine, chlorine or bromine, or
represents a group O-Y,

wherein

Y represents acetyl, methoxymethyl or benzyl,
$R^5$ represents nitro,
represents cyano or
represents the group $CO_2R^9$,

wherein

$R^9$ and $R^4$ together denote a direct bond,
X represents $>C=*$, $-SO_2-$ or -CO-NH-,
$R^6$ represents straight-chain, branched or cyclic alkyl or alkenyl having up to 18 C atoms, which is optionally, substituted by phenyl or one or more fluorine, chlorine, nitro, carboxyl or $C_1$-$C_4$-alkoxycarbonyl,
represents thienyl, furyl, pyrryl, pyridyl, pyrimidyl, pyrazinyl, pyrolyl or pyridazinyl, which are optionally substituted by fluorine, chlorine, bromine or $C_1$-$C_4$-alkyl, or
represents $C_6$-$C_{10}$-aryl which is optionally substituted by up to 4 identical or different substituents from the series comprising fluorine, chlorine, bromine, nitro, $C_1C_6$-alkyl, $C_1$-$C_3$-alkoxy, $C_1$-$C_3$-alkylthio, $C_1$-$C_4$-alkoxycarbonyl, $C_1$-$C_4$-alkylsulphonyl, trifluoromethoxy, trifluoromethylthio, trifluoromethyl or amino, it being possible for this amino group to carry one or two substituents from the series comprising benzoyl, acetyl, phenyl or methyl, or
represents the radical

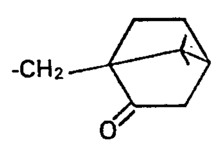

and

$R^7$ and $R^8$ are identical or different and
represent hydrogen,
represent $C_1$-$C_6$-alkoxy,
represent fluorine, chlorine or bromine,
represent $C_1$-$C_6$-alkyl,
represent nitro,
represent cyano, or
represent trifluoromethyl,
and their salts.

2. Compounds according to Claim 1,
in which
$R^1$ represents straight-chain, branched or cyclic alkyl or alkenyl having up to 8 C atoms, which is optionally interrupted by one oxygen or sulphur atom in the chain and is optionally substituted by phenyl, cyano, one or more fluorine or N-benzyl-N-methyl-amino,
$R^2$ represents methyl, hydroxymethyl or ethyl,
represents cyano,
$R^3$ represents hydrogen, or
represents methyl or ethyl

$R^4$ represents hydrogen or hydroxyl,
represents methyl or ethyl,
represents chlorine or bromine, or
represents a group O-Y,

wherein

Y represents acetyl,
$R^5$ represents nitro, or
represents $CO_2R^9$,

wherein

$R^9$ and $R^4$ together denote a direct bond,
X represents $>C=O$, $-SO_2-$ or -CO-NH-,
$R^6$ represents straight-chain, branched or cyclic alkyl or alkenyl having up to 16 C atoms and is optionally substituted by phenyl, or one or more fluorine or chlorine,
represents thienyl, furyl or pyridyl, each of which is optionally substituted by chlorine or methyl or represents phenyl or naphthyl which optionally carries up to 3 identical or different substituents from the series comprising fluorine, chlorine, nitro, $C_1$-$C_4$-alkyl, methoxy, methylthio, trifluoromethoxy, trifluoromethylthio, trifluoromethyl or acetylamino, or
represents the radical

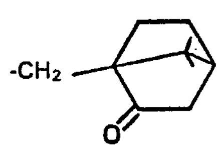

and
$R^7$ and $R^8$ are identical or different and
represent hydrogen,
represent $C_1$-$C_4$-alkoxy,
represent chlorine,
represent $C_1$-$C_4$-alkyl,
represent nitro or
represent trifluoromethyl,

and their salts.

3. Dihydroxypyridines, according to Claim 1 and 2, for controlling circulatory disorders, coronary disorders, cardiac arrhythmias, for the treatment of cardiac insufficiency and/or for influencing the level of blood sugar.

4. Medicament containing as active compound dihydropyridines according to Claims 1 and 2.

5. Process for the preparation of dihydropyridines of the general formula I

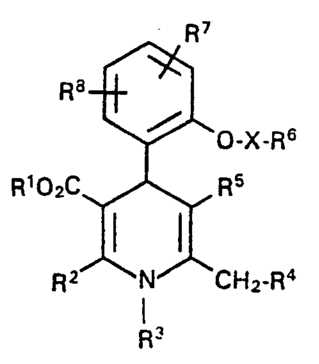

(I)

in which

$R^1$ represents straight-chain, branched or cyclic alkyl or alkenyl having up to 10 C atoms, which is optionally interrupted in the chain by up to 3 oxygen and/or sulphur atoms and is optionally substituited by phenyl, cyano, hydroxyl, one or more fluorine, chlorine, bromine, carboxyl, $C_1$-$C_4$-alkoxycarbonyl, $C_1$-$C_4$-alkylsulphonyl, or by an amino group, it being possible for the amino group to carry one or two identical or different substituents from the group comprising $C_1$-$C_4$-alkyl, phenyl, benzyl, acetyl or benzoyl,
$R^2$ represents straight-chain or branched alkyl having up to 4 C atoms, which is optionally substituted by one or more fluorine, chlorine, bromine, phenyl or hydroxyl, or
represents cyano,
$R^3$ represents hydrogen, or
represents straight-chain or branched alkyl having up to 4 C atoms,
$R^4$ represents hydrogen or hydroxyl,
represents straight-chain or branched alkyl having up to 4 C atoms,
represents fluorine, chlorine or bromine, or
represents a group O-Y,

wherein

Y represents acetyl, methoxymethyl or benzyl,
$R^5$ represents nitro,
represents cyano or
represents the group $CO_2R^9$,

wherein

$R^9$ and $R^4$ together denote a direct bond,
X represents $>C=O$, $-SO_2-$ or -CO-NH-,
$R^6$ represents straight-chain, branched or cyclic alkyl or alkenyl each of which has up to 18 C atoms and is optionally substituted by phenyl, one or more fluorine, chlorine, nitro, carboxyl or $C_1$-$C_4$-alkoxycarbonyl, or
represents thienyl, furyl, pyrryl, pyridyl, pyrimidyl, pyrazinyl, pyrolyl or pyridazinyl, which are optionally substituted by fluorine, chlorine, bromine or $C_1$-$C_4$-alkyl, or
represents $C_6$-$C_{10}$-aryl which is optionally substituted by up to 4 identical or different substituents from the series comprising fluorine, chlorine, bromine, nitro, $C_1$-$C_6$-alkyl, $C_1$-$C_3$-alkoxy, $C_1$-$C_3$-alkylthio, $C_1$-$C_4$-alkyxycarbonyl, $C_1$-$C_4$-alkylsulphonyl, trifluoromethoxy, trifluoromethylthio, trifluoromethyl or amino, it being possible for this amino group to carry one or two substituents from the series comprising benzoyl, acetyl, phenyl or methyl, or
represents the radical

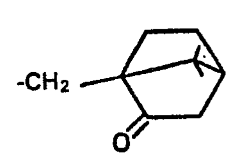

and

$R^7$ and $R^8$ are identical or different and
represent hydrogen,
represent $C_1$-$C_6$-alkoxy,
represent fluorine, chlorine or bromine,
represent $C_1$-$C_6$-alkyl,
represent nitro,
represent cyano, or
represent trifluoromethyl,
and their salts,

but it not being permissible for $R^5$ to represent the radical $CO_2R^9$, characterized in that

[A] aldehydes of the general formula (II)

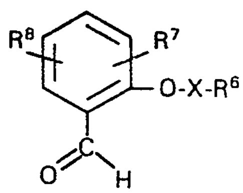

(II)

in which
$R^6$, $R^7$, $R^8$ and X have the abovementioned meaning, and keto compounds of the general formula (III)

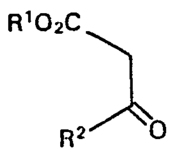

(III)

in which
$R^1$ and $R^2$ have the abovementioned meaning, are reacted with keto compounds of the general formula (IV)

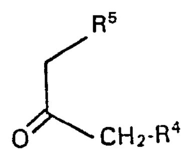

(IV)

in which
$R^4$ and $R^5$ have the abovementioned meaning and amines of the general formula (V)

$R^3$-$NH_2$ (V)

in which
$R^3$ has the abovementioned meaning where appropriate in the presence of water and/or inert organic solvents, or when

[B] aldehydes of the general formula (II) are reacted with keto compounds of the general formula (III) and enamines of the general formula (VI)

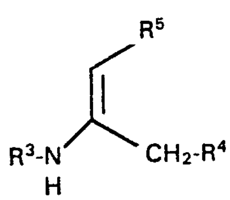

(VI)

in which
$R^3$, $R^4$ and $R^5$ have the abovementioned meaning, where appropriate in the presence of water and/or inert organic solvents,
or when

[C] aldehydes of the general formula (II) are reacted with keto compounds of the general formula (IV) and enamines of the general formula (VII)

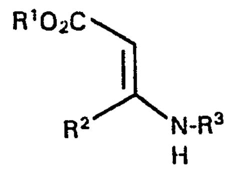

(VII)

in which
$R^1$, $R^2$ and $R^3$ have the abovementioned meaning, where appropriate in the presence of water and/or inert organic solvents,
or when

[D] keto compounds of the general formula (III) are reacted with amines of the general formula (V) and ylidene compounds of the general formula (VIII)

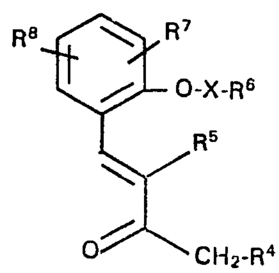

(VIII)

in which
$R^4$, $R^5$, $R^6$, $R^7$, $R^8$ and X have the abovementioned meaning, where appropriate in the presence of water and/or inert organic solvents,
or when

[E] keto compounds of the general formula (IV) are reacted with amines of the general formula (V) and ylidene compounds of the general formula (IX)

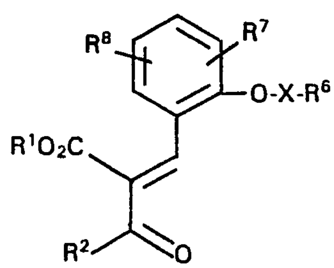

(IX)

in which
$R^1$, $R^2$, $R^6$, $R^7$, $R^8$ and X have the abovementioned meaning, where appropriate in the presence of water and/or inert organic solvents,
or when

[F] ylidene compounds of the general formula (VIII) are reacted with enamines of the general formula (VII), where appropriate in the presence of water and/or inert organic solvents,
or when

[G] ylidene compounds of the general formula (IX) are reacted with enamines of the general formula (VI), where appropriate in the presence of water and/or inert organic solvents.

6. Process for the preparation of dihydropyridines of the general formula I, according to Claim 5, in which
$R^1$, $R^2$, $R^3$, $R^4$, $R^5$, X, $R^6$, $R^7$ and $R^8$ have the meaning given in Claim 5, wherein $R^5$ represents the group $CO_2R^9$, and $R^4$ and $R^9$ together represent a bond, characterized in that

[H] aldehydes of the general formula II

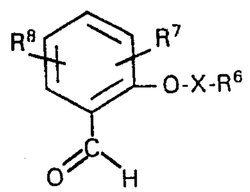
(II),

in which
$R^6$-$R^8$ and X have the abovementioned meaning, are reacted with keto compounds of the general formula III

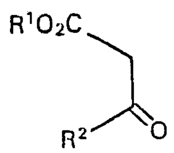
(III)

in which
$R^1$ and $R^2$ have the abovementioned meaning, and with enamines of the formula X

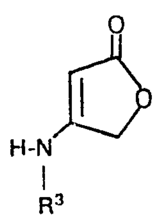
(X)

in which
$R^3$ has the abovementioned meaning, where appropriate in the presence of water and/or inert organic solvents, or in that

[I] dihydropyridines of the general formula XI

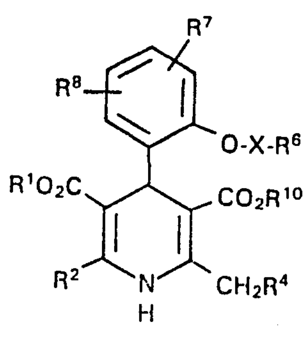
(XI),

in which
$R^4$ represents halogen,
represents a group O-Y,

wherein

Y has the abovementioned meaning, and
$R^{10}$ represents straight-chain or branched alkyl having up to 6 C atoms,

are cyclized, where appropriate in the presence of inert organic solvents and, where appropriate, in the presence of bases, or when $R^4$ represents halogen, are pyrolysed with or without solvents.

7. Process for the preparation of medicaments according to Claim 4, characterized in that the active compounds are mixed with customary auxiliaries and/or vehicles.

8. Use of dihydropyridines of Claims 1 and 2 for the preparation of medicaments for controlling circulatory disorders, coronary disorders, cardiac arrhythmias, for the treatment of cardiac insufficiency and/or for influencing the level of blood sugar.

**Revendications**

1. Dihydropyridines de formule générale (I):

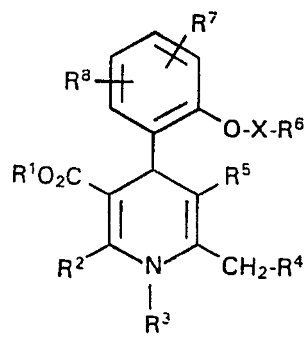
(I)

dans laquelle
$R^1$ représente un groupe alkyle ou alcényle, linéaire, ramifié ou cyclique, ayant jusqu'à 10 atomes de carbone, qui peut éventuellement être interrompu dans la chaîne par 3 atomes d'oxygène et/ou de soufre, et qui est éventuellement substitué par un groupe phényle, cyano, hydroxy, un ou plusieurs atomes de fluor, de chlore ou de brome, un groupe carboxy, alcoxy (en $C_1$-$C_4$)-carbonyle, alkylsulfonyle en $C_1$ à $C_4$ ou par un groupe amino, le groupe amino pouvant porter 1 ou 2 substituants, identiques ou différents, choisis parmi un groupe alkyle en $C_1$ à $C_4$, un groupe phényle, benzyle, acétyle ou benzoyle,
$R^2$ représente un groupe alkyle, linéaire ou ramifié, ayant jusqu'à 4 atomes de carbone, qui est éventuellement substitué par un ou plusieurs atomes de fluor, de chlore ou de brome, par un groupe phényle ou hydroxyle, ou bien $R^2$ représente un groupe cyano,
$R^3$ représente un atome d'hydrogène, ou un groupe alkyle, linéaire ou ramifié, ayant jusqu'à 4 atomes de carbone,
$R^4$ représente un atome d'hydrogène, un groupe

hydroxyle, un groupe alkyle, linéaire ou ramifié, ayant jusqu'à 4 atomes de carbone, un atome de fluor, de chlore ou de brome,
un groupe O-Y, où Y représente un groupe acétyle, méthoxyméthyle ou benzyle,

$R^5$ représente un groupe nitro, un groupe cyano ou le groupe $CO_2R^9$, où $R^9$ et $R^4$ représentent alors ensemble une liaison directe,

X représente >C=O, $-SO_2-$ ou -CO-NH-,

$R^6$ représente un groupe alkyle ou alcényle, linéaire, ramifié ou cyclique, comportant jusqu'à 18 atomes de carbone, qui est éventuellement substitué par un groupe phényle, par un ou plusieurs atomes de fluor, de chlore ou groupes nitro, carboxy ou alcoxy (en $C_1$ à $C_4$)-carbonyle ou bien $R^6$ représente un groupe thiényle, furyle, pyrryle, pyridyle, pyrimidyle, pyrazinyle, pyrrolyle, pyridazinyle (éventuellement substitués par le fluor, le chlore ou le brome ou par un groupe alkyle en $C_1$ à $C_4$), ou $R^6$ représente un groupe aryle en $C_6$ à $C_{10}$, qui est éventuellement substitué par 1 à 4 substituants, identiques ou différents, choisis parmi un atome de fluor, de chlore ou de brome, un groupe nitro, alkyle en $C_1$ à $C_6$, alcoxy en $C_1$ à $C_3$, alkylthio en $C_1$ à $C_3$, alcoxy (en $C_1$ à $C_4$)-carbonyle, alkylsulfonyle en $C_1$ à $C_4$, trifluorométhoxy, trifluorométhylthio, trifluorométhyle ou amino, ce groupe amino pouvant porter 1 ou 2 substituants choisis parmi un groupe benzoyle, acétyle, phényle ou méthyle ou

$R^6$ représente le reste

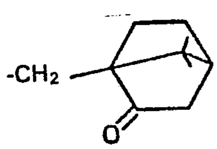

et

$R^7$, $R^8$ sont identiques ou différents et représentent chacun un atome d'hydrogène, un groupe alcoxy en $C_1$ à $C_6$, un atome de fluor, de chlore ou de brome, un groupe alkyle en $C_1$ à $C_6$, un groupe nitro, un groupe cyano ou un groupe trifluorométhyle,

ainsi que leurs sels.

2. Composés selon la revendication 1, dans lesquels

$R^1$ représente un groupe alkyle ou alcényle, linéaire, ramifié ou cyclique, ayant jusqu'à 8 atomes de carbone, qui est éventuellement interrompu dans sa chaîne par 1 atome d'oxygène ou par 1 atome de soufre et qui est éventuellement substitué par un groupe phényle, cyano ou par un ou plusieurs atomes de fluor ou groupes N--benzyl-N-méthylamino,

$R^2$ représente un groupe méthyle, hydroxyméthyle ou éthyle, ou un groupe cyano,

$R^3$ représente un atome d'hydrogène ou bien un groupe méthyle ou éthyle,

$R^4$ représente un atome d'hydrogène ou un groupe hydroxyle, ou bien un groupe méthyle ou éthyle ou un atome de chlore ou de brome ou bien un groupe O-Y, dans lequel Y représente un groupe acétyle,

$R^5$ représente un groupe nitro ou $CO_2R^9$, $R^9$ et $R^4$ signifiant alors ensemble une liaison directe,

X représente >C=O, $-SO_2-$ ou -CO-NH-,

$R^6$ représente un groupe alkyle ou alcényle, linéaire, ramifié ou cyclique ayant jusqu'à 16 atomes de carbone, qui est éventuellement substitué par un groupe phényle ou par un ou plusieurs atomes de fluor ou de chlore, ou bien $R^6$ représente un groupe thiényle, furyle ou pridyle (éventuellement substitué par du chlore ou par un groupe méthyle), ou $R^6$ représente un groupe phényle ou naphtyle, qui porte éventuellement jusqu'à 3 substituants, identiques ou différents, choisi parmi un atome de fluor ou de chlore, un groupe nitro, un groupe alkyle en $C_1$ à $C_4$, méthoxy, méthylthio, trifluorométhoxy, trifluorométhylthio, trifluorométhyle ou acétylamino, ou bien

$R^6$ représente le reste

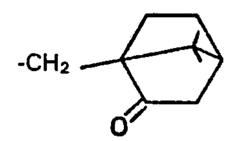

et

$R^7$ et $R^8$ sont identiques ou différents et représentent chacun un atome d'hydrogène, un groupe alcoxy en $C_1$ à $C_4$, un atome de chlore, un groupe alkyle en $C_1$ à $C_4$, un groupe nitro ou un groupe trifluorométhyle,

ainsi que leurs sels.

3. Dihydropyridines selon les revendications 1 et 2, pour combattre des maladies de la circulation sanguine, des maladies coronariennes, des troubles du rythme cardiaque, pour traiter une insuffisance cardiaque et/ou pour influer sur le taux de sucre sanguin.

4. Médicament contenant comme substance active des dihydropyridines selon les revendications 1 et 2.

5. Procédé pour préparer des dihydropyridines de formule générale (I)

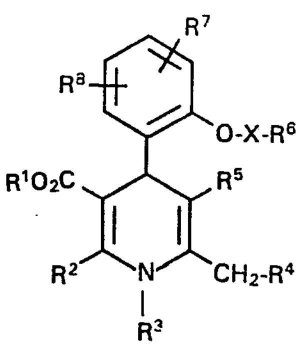

(I)

dans laquelle

$R^1$ représente un groupe alkyle ou alcényle, linéaire, ramifié ou cyclique, ayant jusqu'à 10 atomes de carbone, qui est éventuellement interrompu dans sa chaîne par 3 atomes d'oxygène et/ou de soufre et qui est éventuellement substitué par un groupe phényle, cyano, hydroxy, par un ou plusieurs atomes de fluor, de chlore ou de

brome, par un groupe carboxy, alcoxy (en $C_1$ à $C_4$)-carbonyle, alkylsulfonyle ayant 1 à 4 atomes de carbone ou par un groupe amino, le groupe amino pouvant porter 1 ou 2 substituants, identiques ou différents, choisis parmi un groupe alkyle en $C_1$ à $C_4$, phényle, benzyle, acétyle ou benzoyle,

$R^2$ représente un groupe alkyle, linéaire ou ramifié, ayant jusqu'à 4 atomes de carbone, qui est éventuellement substitué par un ou plusieurs atomes de fluor, de chlore ou de brome ou par des groupes phényles ou hydroxy, ou bien $R^2$ représente un groupe cyano,

$R^3$ représente un atome d'hydrogène, ou un groupe alkyle, linéaire ou ramifié, ayant jusqu'à 4 atomes de carbone,

$R^4$ représente un atome d'hydrogène ou un groupe hydroxy, un groupe alkyle, linéaire ou ramifié, ayant jusqu'à 4 atomes de carbone, un atome de fluor, de chlore ou de brome, ou bien un groupe O-Y, où Y représente un groupe acétyle, méthoxyméthyle ou benzyle,

$R^5$ représente un groupe nitro, un groupe cyano ou le groupe $CO_2R^9$, $R^9$ et $R^4$ signifiant alors ensemble une liaison directe,

X représente $>C=O$, $-SO_2-$ ou -CO-NH-,

$R^6$ représente un groupe alkyle ou alcényle, linéaire, ramifié ou cyclique, ayant jusqu'à 18 atomes de carbone, qui est éventuellement substitué par un groupe phényle, un ou plusieurs atomes de fluor ou de chlore ou groupes nitro, carboxy ou alcoxy (en $C_1$ à $C_4$)-carbonyle ou bien $R^6$ représente un groupe thiényle, furyle, pyrryle, pyridyle, pyrimidyle, pyrazinyle, pyrrolyle, pyridazinyle (éventuellement substitués par du fluor, du chlore ou du brome ou par un groupe alkyle en $C_1$-$C_4$), ou $R^6$ représente un groupe aryle en $C_6$ à $C_{10}$, qui est éventuellement substitué par 4 substituants identiques ou différents, choisis parmi un atome de fluor, de chlore ou de brome, un groupe nitro, un grupe alkyle en $C_1$ à $C_6$, alcoxy en $C_1$ à $C_3$, alkylthio en $C_1$ à $C_3$, alcoxy (en $C_1$ à $C_4$)-carbonyle, alkylsulfonyle en $C_1$ à $C_4$, trifluorométhoxy, trifluorométhylthio, trifluorométhyle ou amino, ce groupe amino pouvant porter 1 ou 2 substituants choisis parmi un groupe benzoyle, acétyle, phényle ou méthyle ou bien

$R^6$ représente le reste 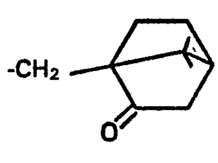

et

$R^7$, $R^8$ sont identiques ou différents et représentent chacun un atome d'hydrogène, un groupe alcoxy en $C_1$ à $C_6$, un atome de fluor, de chlore ou de brome, un groupe alkyle en $C_1$ à $C_6$, un groupe nitro, un groupe cyano ou un groupe trifluorométhyle,

ainsi que leurs sels,

le symbole $R^5$ ne devant cependant pas représenter le reste $CO_2R^9$, procédé caractérisé en ce que

[A] on fait réagir des aldéhydes de formule généraLE (II)

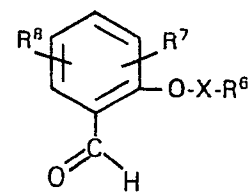

(II),

dans laquelle $R^6$, $R^7$, $R^8$ et X ont le sens précité, et des composés cétoniques de formule générale (III)

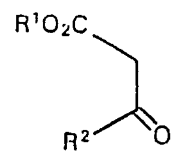

(III)

dans laquelle $R^1$ et $R^2$ ont le sens précité, avec des composés cétoniques de formule générale (IV)

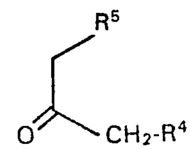

(IV),

dans laquelle $R^4$ et $R^5$ ont le sens précité, et des amines de formule générale (V)

$R^3-NH_2$ (V),

dans laquelle $R^3$ a le sens précité, en opérant éventuellement en présence d'eau et/ou de solvants organiques inertes,

ou

[B] on fait réagir des aldéhydes de formule générale (II) avec des composés cétoniques de formule générale (III) et des énamines de formule générale (VI)

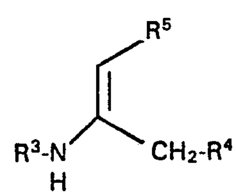

(VI),

dans laquelle $R^3$, $R^4$ et $R^5$ ont le sens précité, un opérant éventuellement en présence d'eau et/ou de solvants organiques inertes,

ou

[C] on fait réagir des aldéhydes de formule générale (II) avec des composés cétoniques de formule générale (IV) et avec des énamines de formule générale (VII)

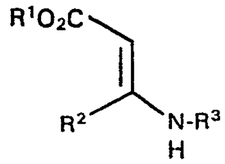

(VII),

dans laquelle $R^1$, $R^2$ et $R^3$ ont le sens précité, en opérant éventuellement en présence d'eau et/ou de solvants organiques inertes,
ou

[D] on fait réagir des composés cétoniques de formule générale (III) avec des amines de formule générale (V) et des composés ylidéniques de formule générale (VIII)

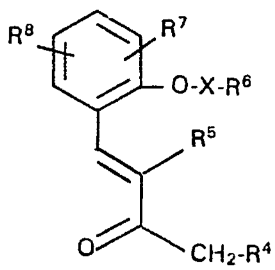

(VIII),

dans laquelle $R^4$, $R^5$, $R^6$, $R^7$, $R^8$ et X ont le sens précité, en opérant éventuellement en présence d'eau et/ou de solvants organiques inertes,
ou bien

[E] on fait réagir des composés cétoniques de formule générale (IV) avec des amines de formule générale (V) et avec des composés ylidéniques de formule générale (IX)

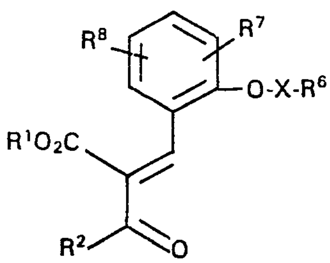

(IX),

dans laquelle $R^1$, $R^2$, $R^6$, $R^7$, $R^8$ et X ont le sens précité, en opérant éventuellement en présence d'eau et/ou de solvants organiques inertes,
ou

[F] on fait réagir des composés ylidéniques de formule générale (VIII) avec des énamines de formule générale (VII), en opérant éventuellement en présence d'eau et/ou de solvants organiques inertes,
ou bien

[G] on fait réagir des composés ylidéniques de formule générale (IX) avec des énamines de formule générale (VI), en opérant éventuellement en présence d'eau et/ou de solvants organiques inertes.

6. Procédé pour préparer des dihydropyridines répondant à la formule générale (I) selon la revendication 5, dans laquelle $R^1$, $R^2$, $R^3$, $R^4$, $R^5$, X, $R^6$, $R^7$ et $R^8$ ont le sens indiqué à la revendication 5, le symbole $R^5$ représentant le groupe $CO_2R^9$ et $R^4$ et $R^9$ formant ensemble une liaison, procédé caractérisé en ce que

[H] on fait réagir des aldéhydes de formule générale (II)

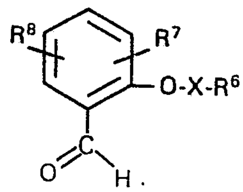

(II),

dans laquelle $R^6$-$R^8$ et X ont le sens précité, avec des composés cétoniques de formule générale (III)

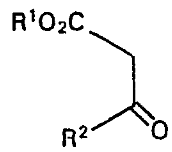

(III),

dans laquelle $R^1$ et $R^2$ ont le sens précité, avec des énamines de formule (X)

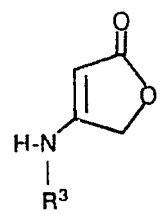

(X),

dans laquelle $R^3$ a le sens précité, en opérant éventuellement en présence d'eau et/ou de solvants organiques inertes,
ou

[I] on soumet des dihydropyridines de formule générale (XI)

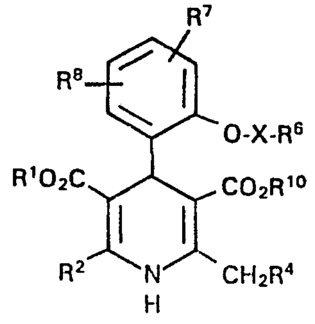

(XI),

dans laquelle
$R^4$ représente un atome d'halogène, ou un groupe O-Y, le symbole Y ayant le sens précité,

et

$R^{10}$ représente un groupe alkyle, linéaire ou ramifié, ayant jusquà 6 atomes de carbone,

à cyclisation, en opérant éventuellement en présence de bases,

ou bien

lorsque $R^4$ représente un halogène, on soumet à pyrolyse avec ou sans solvant.

7. Procédé pour préparer des médicaments selon la revendication 4, caractérisé en ce qu'on mélange les substances actives avec des adjuvants et/ou des supports ou excipients usuels.

8. Utilisation des dihydropyridines selon les revendications 1 et 2 pour préparer des médicaments destinés à combattre des maladies de la circulation du sang, des maladies coronariennes, des troubles du rythme cardiaque, pour traiter une insuffisance cardiaque et/ou pour influer sur le taux de sucre sanguin.